(19) Europäisches Patentamt
European Patent Office
Office européen des brevets

(11) EP 4 729 617 A1

(12) # EUROPEAN PATENT APPLICATION
published in accordance with Art. 153(4) EPC

(43) Date of publication:
**22.04.2026 Bulletin 2026/17**

(21) Application number: **23941743.9**

(22) Date of filing: **02.11.2023**

(51) International Patent Classification (IPC):
***C12N 15/115*** *(2010.01)* ***A61K 47/54*** *(2017.01)*
***A61P 35/00*** *(2006.01)*

(52) Cooperative Patent Classification (CPC):
**A61K 47/54; A61P 35/00; C12N 15/115**

(86) International application number:
**PCT/KR2023/017344**

(87) International publication number:
**WO 2024/257962 (19.12.2024 Gazette 2024/51)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **16.06.2023 KR 20230077743**

(71) Applicant: **Aptamer Sciences Inc.**
**Seongnam-si, Gyeonggi-do 13487 (KR)**

(72) Inventors:
- **LEE, Daekyun**
  **Yongin-si, Gyeonggi-do 16919 (KR)**
- **KANG, Ju-hyung**
  **Incheon 22535 (KR)**
- **LIM, Soryong**
  **Yongin-si, Gyeonggi-do 16963 (KR)**
- **SONG, Inu**
  **Seongnam-si, Gyeonggi-do 13632 (KR)**
- **RYU, Hwarim**
  **Seongnam-si, Gyeonggi-do 13438 (KR)**

(74) Representative: **Icosa**
**83 avenue Denfert-Rochereau**
**75014 Paris (FR)**

Remarks:
The complete document including Reference Table(s) and the Sequence Listing(s) can be downloaded from the EPO website

(54) **APTAMER TARGETING CD25, DRUG CONJUGATE COMPRISING SAME, AND USE THEREOF**

(57) The present invention relates to an aptamer targeting CD25, a drug conjugate comprising the same, and use thereof

[FIG. 1]

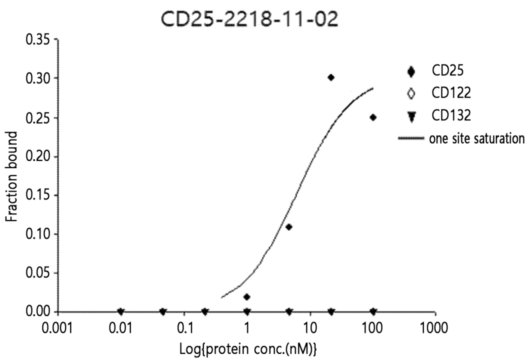


| | CD25 | CD122 | CD132 |
|---|---|---|---|
| Kd(nM) | 6.20 | - | - |
| Bmax | 0.31 | - | - |



Processed by Luminess, 75001 PARIS (FR)

## Description

### [Technical Field]

The present invention relates to an aptamer targeting CD25, a drug conjugate comprising the same, and use thereof.

### [Background Art]

Cancer immunotherapy is an anticancer treatment that has a therapeutic approach to activating immune cells through enhancement of a patient's own immune system, thereby killing cancer cells, and is applicable to various types of carcinomas and has the advantage of exhibiting sustained anticancer effects because it utilizes the immune system's memory and adaptive capabilities.

Tumor-infiltrating regulatory T cells (regulatory T lymphocytes, Tregs), which are a primary target for such cancer immunotherapy, limit the intratumoral infiltration of effector T cells (effector T lymphocytes, Teffs) and significantly contribute to the immune evasion of cancer cells. A high ratio of Treg/Teff is closely associated with cancer prognosis and limited therapeutic efficacy of cancer immunotherapies (e.g., immune checkpoint inhibitors, *etc.).* Accordingly, when selective depletion of intratumoral Tregs is combined with existing cancer immunotherapy regimens, a more effective anti-cancer effect may be expected.

However, targeting Tregs may affect not only intratumoral Tregs but also systemic Tregs, raising concerns about autoimmune-related side effects. Therefore, there is a need to develop a technology that minimizes the impact on systemic Tregs and specifically eliminates only tumor-infiltrating Tregs, thereby increasing the proportion of Teffs. The cytokine interleukin-2 (IL-2) is essential for the development and function of Tregs. Although Tregs cannot produce IL-2 autonomously, they constitutively express CD25 (IL-2R$\alpha$), the alpha subunit of its receptor, at high levels on the cell surface. In contrast, CD25 is absent or expressed at low levels on the surface of Teff cells. Therefore, CD25 is one of the potential molecules capable of specifically targeting and depleting Tregs. In addition, CD25 is highly expressed in numerous hematologic malignancies, such as adult T-cell leukemia, acute myeloid leukemia, acute lymphoblastic leukemia, Hodgkin's lymphoma, non-Hodgkin's lymphoma, *etc.*

In this regard, previous studies demonstrated the potential for selective depletion of Tregs through targeting CD25 using anti-murine CD25 antibodies (PC61 clone) in a nonclinical animal model (Y. Y. Setiady et al., Eur. J. Immunol., 2010, 40(3): 780-786), and radioimmunotherapy for CD25-positive leukemia was studied using radionuclide-labeled anti-human CD25 antibodies (7G7/B6 clone) in a nonclinical animal model (M. Zhang. et al., Cancer Res., 2006, 66(16): 8227, *etc.).*

Meanwhile, an aptamer is a single-stranded nucleic acid (DNA, RNA, or modified nucleic acid) characterized by having a stable tertiary structure in itself and being capable of binding to a target molecule with high affinity and specificity. Since the first development of an aptamer discovery technology called Systematic Evolution of Ligands by EXponential enrichment (SELEX) in 1990, many aptamers capable of binding to various target molecules ranging from low-molecular-weight organic substances and peptides to membrane proteins have been continuously discovered. The aptamer is often comparable to a monoclonal antibody due to its characteristic of binding to a target molecule with unique high affinity (usually at the pM level), and has high potential as an alternative to antibodies, especially also called a "chemical antibody". In particular, since aptamers have a shorter half-life compared to antibodies, targeting Tregs using aptamers minimizes the effect on systemic Tregs, thereby reducing the risk of autoimmune side effects.

### [Disclosure]

### [Technical Problem]

The present inventors have developed a CD25-targeting aptamer and a drug conjugate thereof that have a therapeutic effect in cancer immunotherapy employing a mechanism that inhibits the binding between IL-2 and CD25 (IL-2R$\alpha$), the alpha subunit of its receptor, thereby suppressing the activation of CD25-positive T cells while inducing selective depletion of tumor-infiltrating Tregs, and/or enable the treatment of tumors associated with CD25-positive hematologic malignancies, thereby completing the present invention.

### [Technical Solution]

An object of the present invention is to provide a CD25 aptamer specifically binding to CD25 protein.

Another object of the present invention is to provide a CD25 aptamer-drug conjugate (CD25 ApDC) comprising the CD25 aptamer, and one or more drugs conjugated to the CD25 aptamer.

Still another object of the present invention is to provide a pharmaceutical composition for treating cancer diseases, the pharmaceutical composition comprising the CD25 aptamer or CD25 aptamer-drug conjugate as an active ingredient.

Still another object of the present invention is to provide a method of treating cancer diseases, the method comprising the step of administering the pharmaceutical composition to a subject in need thereof.

**[Advantageous Effects]**

An aptamer of the present invention specifically binds to CD25 protein, thereby inhibiting binding of IL-2 and CD25 protein and effectively suppressing activation of CD25-positive T cells. Furthermore, a drug conjugate based on this aptamer has a shorter half-life compared to antibodies, thereby minimizing side effects, and thus it may be usefully applied to cancer immunotherapy inducing the selective depletion of tumor-infiltrating Tregs and/or to the treatment of tumors associated with CD25-positive hematologic malignancies.

**[Brief Description of the Drawing]**

FIG. 1 shows the results of confirming the binding potential of an aptamer of the present invention to rhCD25 (IL-2Rα), rhCD122 (IL-2Rβ), and rhCD132 (IL-2Rγ) proteins which are IL-2 receptor proteins;

FIG. 2 shows the results of confirming the binding potential of an aptamer of the present invention to rhCD25 (IL-2Rα), rhCD122 (IL-2Rβ), and rhCD132 (IL-2Rγ) proteins which are IL-2 receptor proteins;

FIG. 3 shows the results of confirming the binding potential of an aptamer of the present invention to rhCD25 (IL-2Rα), rhCD122 (IL-2Rβ), and rhCD132 (IL-2Rγ) proteins which are IL-2 receptor proteins;

FIG. 4 shows the results of confirming the binding potential and internalization of the aptamers of the present invention to CD25-positive cells or CD25-negative cells;

FIG. 5 shows the results of confirming the inhibitory effects of the aptamers of the present invention on binding of IL-2/IL-2Rα by competitive ELISA;

FIG. 6 shows the results of confirming IL-2-stimulated pSTAT5 levels in cells treated with the aptamer of the present invention;

FIG. 7 illustrates secondary and tertiary structures of the aptamers of the present invention;

FIG. 8 shows the results of confirming the binding potential of the aptamers of the present invention to a human CD25 protein;

FIG. 9 shows the results of confirming the binding potential of the aptamers of the present invention to CD25-positive cells or CD25-negative cells;

FIG. 10 shows the results of confirming internalization of the aptamers of the present invention into CD25-positive cells or CD25-negative cells;

FIG. 11 shows the results of confirming the internalization rate of the aptamers of the present invention into CD25-positive cells;

FIG. 12 shows the results of confirming the inhibitory effects of the aptamers of the present invention on binding of IL-2/IL-2Rα by competitive ELISA;

FIG. 13 shows the results of confirming IL-2-stimulated TGF-β levels in cells treated with the aptamer of the present invention;

FIG. 14 illustrates a secondary structure of the chemically-optimized CD25 combi aptamer of the present invention;

FIG. 15 shows the results of confirming the plasma stability of the chemically-optimized CD25 combi aptamer of the present invention;

FIG. 16 shows the results of confirming internalization of the aptamer of the present invention via the endosome-lysosome pathway;

FIG. 17 illustrates a synthesis scheme for an aptamer-drug conjugate (DApR=1) of the present invention;

FIG. 18 illustrates a synthesis scheme for an aptamer-drug conjugate (DApR=3) of the present invention;

FIG. 19 shows the results of confirming induction of CD25-positive cell-specific death by the aptamer-drug conjugates of the present invention; and

FIG. 20 shows the results of confirming induction of Treg cell-specific death by the aptamer-drug conjugates of the present invention.

**[Detailed Description of Preferred Embodiments]**

Each description and embodiment disclosed in this disclosure may also be applied to other descriptions and

embodiments. That is, all combinations of various elements disclosed in this disclosure fall within the scope of the present invention. Further, the scope of the present invention is not limited by the specific description described below.

Further, those skilled in the art will recognize, or be able to ascertain using no more than routine experimentation, many equivalents to the specific embodiments of the disclosure described herein. Further, these equivalents should be interpreted to fall within the present invention.

Furthermore, throughout this specification, when a part is referred to as "comprising" an element, it is understood that other elements may be further comprised rather than other elements being excluded unless content to the contrary is specially described.

Hereinafter, the present invention will be described in more detail.

A first aspect of the present invention provides a CD25 aptamer specifically binding to CD25 protein.

For example, the aptamer may comprise one nucleotide sequence selected from the group consisting of the following General Formulae 1 to 3 or a polynucleotide sequence having 80% or more homology or identity thereto:

[General Formula 1] TTCGAGATCAAGTTATAGCGTGTCCCATTCTATCCAAGACACT (SEQ ID NO: 1)

[General Formula 2] GTGTGTGTCACTCTATCGTTACGAGGAATACGGGCAAAGC (SEQ ID NO: 2)

[General Formula 3] CGGTGCAATCGCGTGGTCAACATCCTGTTAACCTCGGAAT (SEQ ID NO: 3).

As used herein, the term "aptamer", also referred to as a "chemical antibody", means a short, single-stranded nucleic acid molecule (composed of 20 bases to 120 bases) that possesses the characteristic of being able to bind with high specificity and affinity to various types of target ligands, ranging from specific compounds to proteins. Aptamers may be developed *in vitro* through systematic evolution of ligands by exponential enrichment (SELEX).

Aptamers are considered oligonucleotide molecules that share characteristics similar to antibodies in terms of possessing high binding potential and selectivity toward a target protein at the nanomolar (nM) to picomolar (pM) level. Meanwhile, compared to antibodies, aptamers have advantages in that (1) they are prepared by chemical synthesis, are easily subjected to desired chemical modification, and have high stability compared to protein-based materials such as antibodies; (2) their selectivity and affinity may be maximized through the SELEX process; (3) they have high purity because they are produced by chemical synthesis; and (4) ultimately, quality control of the finally produced material is easy.

As used herein, the term "systematic evolution of ligands by exponential enrichment (SELEX)" is a method of selecting aptamers that bind to a target substance. According to a method generally known, the method may be performed by repeating a series of processes of reacting a target protein with an oligonucleotide library (DNA or RNA), which has a random nucleotide sequence, at a predetermined temperature, removing the DNA/RNA library not binding to the target, and isolating the nucleotides binding to the target, and amplifying the same using a gene amplification method, thereby selecting aptamers having high binding potential for the target. The aptamers of the present invention may be prepared using the aforementioned general SELEX method, but are not limited thereto.

Specifically, the aptamer selection process generally requires a process of obtaining a pool of single-stranded nucleic acids from a library with approximately $10^{14}$ to $10^{15}$ different sequences, *i.e.*, diversity. A number of methods are employed for this method, but the most commonly used methods involve a method of amplifying only one strand using asymmetric PCR, and a method of attaching biotin to the end of one strand of double-stranded nucleic acids, and then selectively isolating only one strand using streptavidin-coated beads.

Thereafter, a selection process is carried out, in which the obtained library is bound to the target molecule to sort out aptamers with high binding potential. When the target molecule is a protein, biotin labeled on the protein is generally pulled down using streptavidin beads. After inducing binding between the target protein and the modified nucleic acid library, the modified nucleic acid libraries not bound to the target protein are removed by washing with a buffer.

Similarly, in the case of a plate, binding between the nucleic acid library and the target protein is induced, followed by washing with a buffer to remove nucleic acids not bound to the target protein. By using these methods, aptamers having affinity for the ligand may be obtained. Generally, when the selection-amplification process is repeated 5 times to 15 times, aptamers with high affinity may be obtained. Once the selection process is complete, the amplified nucleic acids are cloned, and then their sequences are identified from individual clones through sequence analysis, and the aptamers are then synthesized to measure their affinity and binding potential for the target molecule.

The "target molecule" may refer to a substance that may be specifically detected using the selected aptamer. Specifically, the target molecule may be one or more selected from the group consisting of proteins, peptides, carbohydrates, polysaccharides, glycoproteins, hormones, receptors, antigens, antibodies, viruses, cofactors, drugs, dyes,

growth factors, and controlled substances which are present in an isolated sample and to which the capture aptamer is able to bind. With respect to the objects of the present invention, the target molecule may be CD25 protein.

The aptamer of the present invention may further comprise 1 nucleotide to 30 nucleotides at any one or more of the 5'- and 3'-ends thereof.

For example, the aptamer of the present invention may comprise any one polynucleotide of the aforementioned General Formulae 1 to 3 and the aforementioned additional nucleotide at the 5'-end, 3'-end, or both ends thereof, and thus the aptamer of the present invention may comprise 41 nucleotides to 103 nucleotides.

The aptamer of the present invention may comprise any one or more polynucleotide sequences selected from the group consisting of the polynucleotide sequences of General Formula 1 (SEQ ID NO: 1), General Formula 2 (SEQ ID NO: 2), and General Formula 3 (SEQ ID NO: 3), or a polynucleotide sequence having 80%, 85%, 90%, 95%, 96%, 97%, 98%, or 99% or more homology or identity thereto. In addition, it is obvious that a polynucleotide consisting of a nucleotide sequence in which some sequences are deleted, modified, substituted, or added is also comprised within the scope of the aptamer of the present invention, as long as it has such homology or identity and exhibits efficacy corresponding to the aptamer.

As used herein, the term 'homology' or 'identity' refers to a degree of relevance between two given nucleotide sequences, and may be expressed as a percentage. The terms homology and identity may often be used interchangeably with each other.

The sequence homology or identity of conserved polynucleotides may be determined by standard alignment algorithms and may be used with a default gap penalty established by the program being used. Substantially, homologous or identical sequences may generally hybridize under moderately or highly stringent conditions along the full length of the sequence or at least about 50%, about 60%, about 70%, about 80%, or about 90% of the full-length. Polynucleotides that comprise degenerate codons instead of codons are also considered in hybridization.

Whether any two polynucleotide sequences have homology, similarity, or identity may be, for example, determined using a known computer algorithm such as the "FASTA" program using default parameters, as in Pearson et al. (PNAS, 1988, 85(8): 2444-2448). Alternatively, it may be determined using the Needleman Wunsch algorithm (Needleman and Wunsch, J. Mol. Biol., 1970, 48: 443-453), as performed using the Needleman program of the EMBOSS package (EMBOSS: The European MolecuLar Biology Open Software Suite, Rice et al., Trends Genet., 2000, 16: 276-277) (version 5.0.0 or later) (GCG program package (Devereux et al., Nucleic Acids Research, 1984, 12: 387-395), BLASTP, BLASTN, FASTA (Atschul et al., J. Mol. Biol., 1990, 215(3): 403-410; Guide to Huge Computers, Martin J. Bishop, Academic Press, San Diego, 1994 and Carillo et al., SIAM J. Appl. Math., 1988, 48(5): 1073-1082). For example, the homology, similarity, or identity may be determined using BLAST or ClustalW of the National Center for Biotechnology Information.

The homology, similarity, or identity of polynucleotides may be, for example, determined by comparing sequence information using the GAP computer program, such as Needleman et al. (J Mol Biol. 1970, 48(3): 443-453), as disclosed in Smith and Waterman (Adv. Appl. Math., 1981, 2(4): 482-489). Briefly, the GAP program defines homology, similarity, or identity as the value obtained by dividing the number of similarly aligned symbols (i.e., nucleotides) by the total number of symbols in the shorter of the two sequences. Default parameters for the GAP program may comprise (1) a binary comparison matrix (comprising a value of 1 for identities and 0 for non-identities) and the weighted comparison matrix of Gribskov et al. (Nucl. Acids Res., 1986, 14(16): 6745-6763), as disclosed in Atlas Of Protein Sequence And Structure (National Biomedical Research Foundation, 1979, pp. 353-358) of Schwartz and Dayhoff (or EDNAFULL substitution matrix (EMBOSS version of NCBI NUC4.4)); (2) a penalty of 3.0 for each gap and an additional 0.10 penalty for each symbol in each gap (or a gap opening penalty of 10 and gap extension penalty of 0.5); and (3) no penalty for end gaps.

Further, whether any two polynucleotide sequences have homology, similarity, or identity with each other may be identified by comparing the sequences in a Southern hybridization experiment under stringent conditions as defined, and appropriate hybridization conditions defined are within the skill of the art, and may be determined by a method well known to those skilled in the art (*e.g.*, J. Sambrook et al., MolecuLar Cloning, A Laboratory Manual, 2nd Edition, Cold Spring Harbor Laboratory press, Cold Spring Harbor, New York, 1989; F.M. Ausubel et al., Current Protocols in MolecuLar Biology, John Wiley & Sons, Inc., New York).

Further, the aptamer of the present invention may comprise any one or more modifications selected from the following modifications in one or more nucleotides that constitute its polynucleotide sequence:

i) substitution of the C5 position of one or more T bases in the nucleotides with one or more selected from the group consisting of a benzyl group, a naphthyl group, a 2-naphthyl group, a pyrrolebenzyl group, and tryptophan;
ii) substitution of the -OH group at the C2 position in one or more nucleotides with one or more selected from the group consisting of deoxy, methoxy, amino, and fluoro (F); and
iii) substitution of one or more T bases in the nucleotides with $U_{Me}$.

For example, the substitution of the C5 position of a T base with a benzyl group may be substitution with 5'-(N-benzylcarboxyamide)-2'-deoxyuridine (Bn-dU), but is not limited thereto.

For another example, the substitution of the C5 position of a T base with a naphthyl group may be substitution with 5-(N-1-naphthylmethylcarboxyamide)-2'-deoxyuridine (NapdU), but is not limited thereto.

For still another example, the substitution of the C5 position of a T base with a 2-naphthyl group may be substitution with 5-(N-(2-naphthylmethyl)carboxyamide)-2'-deoxyuridine (2NapdU), but is not limited thereto.

The NapdU may be a substituent represented by the following Chemical Formula 1:

[Chemical Formula 1]

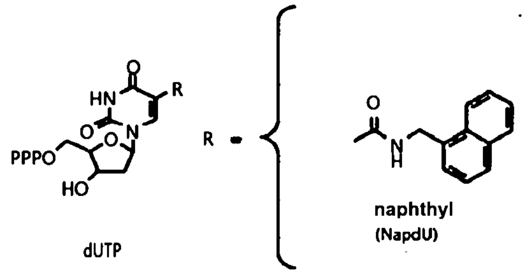

In one embodiment, the aptamer of the present invention comprises the nucleotide sequence of General Formula 1, and the aptamer comprising the aforementioned modification may comprise modifications of any one or more nucleotides selected from the group consisting of nucleotides corresponding to positions 8, 13, 14, 16, 21, 23, 28, 29, 31, 33, and 43 of the nucleotide sequence of General Formula 1, for example, modifications of any one, 2 or more, 3 or more, 4 or more, 5 or more, 6 or more, 7 or more, 8 or more, 9 or more, 10 or more, or 11 nucleotides selected from the aforementioned positions. Specifically, the aptamer of the present invention may comprise any one or more polynucleotide sequences selected from the group consisting of polynucleotide sequences of SEQ ID NOS: 6, 18, and 19.

In another embodiment, the aptamer of the present invention comprises the nucleotide sequence of General Formula 2, and the aptamer comprising the aforementioned modification may comprise modifications of any one or more nucleotides selected from the group consisting of nucleotides corresponding to positions 2, 4, 6, 8, 12, 14, 16, 19, 20, and 29 of the nucleotide sequence of General Formula 2, for example, modifications of any one, 2 or more, 3 or more, 4 or more, 5 or more, 6 or more, 7 or more, 8 or more, 9 or more, or 10 nucleotides selected from the aforementioned positions. Specifically, the aptamer of the present invention may comprise a polynucleotide sequence of SEQ ID NO: 4.

In still another embodiment, the aptamer of the present invention comprises the nucleotide sequence of General Formula 3, and the aptamer comprising the aforementioned modification may comprise modifications of any one or more nucleotides selected from the group consisting of nucleotides corresponding to positions 4, 9, 14, 17, 23, 26, 28, 29, 34, and 40 of the nucleotide sequence of General Formula 3, for example, modifications of any one, 2 or more, 3 or more, 4 or more, 5 or more, 6 or more, 7 or more, 8 or more, 9 or more, or 10 nucleotides selected from the aforementioned positions. Specifically, the aptamer of the present invention may comprise a polynucleotide sequence of SEQ ID NO: 5.

As used herein, the term "corresponding position" refers to a base (nucleotide) at the position recited in a base sequence or polynucleotide, or a nucleotide which is similar, identical, or homologous to the base recited in the base sequence or polynucleotide. As used herein, the "corresponding region" generally refers to a similar or corresponding position in a related polynucleotide or reference polynucleotide.

In the present invention, specific numbering may be used for the base positions within the base sequence or polynucleotide used herein. For example, it is possible to renumber the positions corresponding to the base positions of the polynucleotide of the present invention by aligning a subject nucleotide sequence to be compared with the nucleotide sequence of the present invention.

In the present invention, the aptamer comprising the aforementioned modification may comprise, for example, any one or more polynucleotide sequences selected from the group consisting of polynucleotide sequences of SEQ ID NOS: 4, 5, 6, 18, 19, and 27, or a polynucleotide sequence having 90% or more homology thereto, but is not limited thereto.

In one embodiment of the present invention, a CD25 protein-specific aptamer comprising the nucleotide sequence of General Formula 1 may comprise any one or more polynucleotide sequences selected from the group consisting of polynucleotide sequences of SEQ ID NOS: 6, 18, 19, and 27.

In another embodiment of the present invention, a CD25 protein-specific aptamer comprising the nucleotide sequence of General Formula 2 may comprise any one or more polynucleotide sequences selected from the group consisting of the polynucleotide sequence of SEQ ID NO: 4.

In still another embodiment of the present invention, a CD25 protein-specific aptamer comprising the nucleotide sequence of General Formula 3 may comprise any one or more polynucleotide sequences selected from the group consisting of the polynucleotide sequence of SEQ ID NO: 5.

For example, the substitution of the -OH group at the C2 position in the nucleotide may be, but is not limited to, a substitution with methoxy. The substitution may be achieved by additionally introducing methoxy-substituted uracil ($U_{Me}$), guanine ($G_{Me}$), or both thereof at one end or both ends of the given aptamer sequence, but is not limited thereto. For example, the substitution of the -OH group at the C2 position in the nucleotide may overcome the shortcoming of aptamers, which makes it difficult to develop the aptamers as therapeutic agents due to their short half-life in plasma. In a specific example of the present invention, a chemically optimized combi aptamer was prepared by introducing methoxy-substituted uracil ($U_{Me}$) and guanine ($G_{Me}$) each at both ends of the CD25 aptamer of the present invention, and it was confirmed that a half-life of the aptamer was increased 2.8 times to 8.8 times, as compared to that of the aptamer before optimization, suggesting that significantly improved plasma stability may be achieved through the substitutions.

As described above, the substitution of the -OH group at the C2 position in the nucleotide may provide aptamers with improved functions, such as improved plasma stability, *etc.,* by introducing substitution with methoxy, and such aptamers may be named "chemically-optimized aptamer", "CD25 combi aptamer", *etc.*

The aptamer of the present invention is characterized by specifically binding to CD25 protein. Specifically, the aptamer of the present invention may act as an IL-2 antagonist by binding to CD25 protein. More specifically, the aptamer of the present invention may exert effects such as inhibiting the binding between CD25 and IL-2, inhibiting signal transduction by the binding between CD25 and IL-2, reducing the level of STAT5 phosphorylation by the binding between CD25 and IL-2, or reducing the level of TGF-β by the binding between CD25 and IL-2. Furthermore, the aptamer of the present invention may suppress activation of CD25-positive T cells.

As used herein, the term "CD25" refers to the receptor subunit of interleukin-2 (IL-2) which is a cytokine. The IL-2 receptor subunit is composed of an alpha subunit (IL-2Rα), a beta subunit (IL-2Rβ), and a gamma subunit (IL-2Rγ), and CD25 corresponds to the alpha subunit, IL-2Rα among them. The CD25 may be used interchangeably with CD25 protein or IL-2Rα.

Tumors-infiltrating regulatory T cells (Treg), which are a primary target for cancer immunotherapy, limit the intratumoral infiltration of effector T cells (effector T lymphocytes, Teffs) and significantly contribute to the immune evasion of cancer cells. A high ratio of Treg/Teff is closely associated with cancer prognosis and limited therapeutic efficacy of cancer immunotherapies (*e.g.*, immune checkpoint inhibitors, *etc.).* Accordingly, when the selective depletion of intratumoral Tregs is combined with existing cancer immunotherapy regimens, a more effective anticancer effect may be expected.

However, targeting Tregs may affect not only intratumoral Tregs but also systemic Tregs, raising concerns about autoimmune-related side effects. Therefore, there is a need to develop a technology that minimizes the impact on systemic Tregs and specifically eliminates only tumor-infiltrating Tregs, thereby increasing the proportion of Teffs. IL-2 is essential for the development and function of Tregs. Although Tregs cannot produce IL-2 autonomously, they constitutively express CD25 (IL-2Rα), corresponding to the alpha subunit of its receptor, at high levels on the cell surface. In contrast, CD25 is absent or expressed at low levels on the surface of Teff cells. Therefore, CD25 is one of the potential molecules capable of specifically targeting Tregs. In addition, CD25 is highly expressed in numerous hematologic malignancies, such as adult T-cell leukemia, acute myeloid leukemia, acute lymphoblastic leukemia, Hodgkin's lymphoma, non-Hodgkin's lymphoma, *etc.*

In this regard, previous studies demonstrated the potential for selective depletion of Tregs through targeting CD25 using anti-murine CD25 antibodies (PC61 clone) in a nonclinical animal model (Setiady et al., Eur. J. Immunol., 2010, 40(3): 780-786), and radioimmunotherapy for CD25-positive leukemia was studied using radionuclide-labeled anti-human CD25 antibodies (7G7/B6 clone) in a nonclinical animal model (Zhang. et al., Cancer Res., 2006, 66(16): 8227, *etc.).*

Therefore, the CD25 aptamer of the present invention may specifically bind to CD25 to inhibit the binding of IL-2 to CD25 (IL-2Rα), which corresponds to the alpha subunit of its receptor, thereby suppressing the activation of CD25-positive T cells while inducing the selective depletion of tumor-infiltrating Tregs, and therefore, it enables the therapeutic effects of cancer immunotherapy and/or tumor treatment related to CD25-positive hematologic malignancies.

A second aspect of the present invention provides a CD25 aptamer-drug conjugate (CD25 ApDC) comprising the CD25 aptamer of one aspect and one or more drugs conjugated to the CD25 aptamer.

As used herein, the term "CD25 aptamer of one aspect" is as described above.

For example, in the CD25 aptamer-drug conjugate, the aptamer may further comprise any one or more selected from the group consisting of polyethylene glycol (PEG), hexaethylene glycol (HEG), inverted deoxythymidine (idT), a DBCO linker, an azide linker, an aldehyde linker, an NHS linker, a maleimide linker, a COOH linker, biotin, a fluorescent substance, an amine linker, a thiol linker, cholesterol, and a fatty acid at either one end thereof or at any position in its polynucleotide sequence for conjugation to the drug.

For example, the drug may be an anti-tumor agent. Specifically, the drug may be, but is not limited to, monomethyl auristatin E (MMAE), monomethyl auristatin F (MMAF), cytarabine, gemcitabine, carboplatin, cisplatin, crizotinib, cyclophosphamide, docetaxel, doxorubicin, erlotinib, etoposide, fluorouracil, imatinib mesylate, irinotecan, methotrexate, paclitaxel, sorafenib, sunitinib, topotecan, trabectedin, vincristine, vinblastine, maytansine, DM1, DM4, calicheamicin and

derivatives thereof, doxorubicin, duocarmycin and derivatives thereof, pyrrolobenzodiazepine (PBD), SN-38, α-amanitine, or tubulysin analogs.

For example, the drug may be conjugated to one end of the CD25 aptamer via a chemical linker, conjugated onto the aptamer through interaction between the CD25 aptamer and the drug, or substituted onto the nucleotides constituting the CD25 aptamer.

For example, the drug may be conjugated to any one of both ends of the CD25 aptamer or onto the nucleotides constituting the aptamer via any one or more chemical bonds of covalent, ionic, metallic, van der Waals, and hydrogen bonds, or may be inserted between two or more nucleotides constituting the aptamer, but is not limited thereto.

In this regard, the aptamer and the drug may be in a form where each molecule is conjugated one by one at a 1:1 ratio, or where multiple drug molecules are conjugated to a single aptamer molecule. The number of drug molecules that may be conjugated to a single aptamer molecule may range from 1 to 3, but is not limited thereto, and may vary depending on the method of conjugating the drug to the aptamer. For example, when the drug is conjugated to one end of the CD25 aptamer via a chemical linker, the number of drugs that may be conjugated may vary depending on the type of linker, and for example, 1 drug molecule to 3 drug molecules may be conjugated, but is not limited thereto.

For example, the chemical linker may comprise a combination of a first linker selected from the group consisting of 5'-thiol-modifier C6, thiol-modifier C6 S-S, dithiol serinol, PC amino-modifier, 5'-amino-modifier C3, 5'-amino-modifier C6, 5'-amino-modifier C12, 5'-amino-modifier TEG, amino-modifier TEG, amino-modifier C2 dT, amino-modifier C6 dT, S-Bz-thiol-modifier C6-dT, phosphodiester bond and nucleotides, and a second linker selected from the group consisting of maleimidocaproyl-valine-citrulline-p-aminobenzoyloxycarbonyl (MC-Val-Cit-PAB), hydrazone, peptide, disulfide, thioether, valine-citrulline, N-maleimidomethyl cyclohexane-1-carboxylate (MCC), maleimidocaproyl, mercaptoacetamidocaproyl, N-succinimidyl 4-(2-pyridyldithio)pentanoate (SPP), succinimidyl 4-(N-maleimidomethyl)cyclohexane-1-carboxylate (SMCC), N-succinimidyl 4-(2-pyridylthio)pentanoate (SPDB), phosphodiester bond and nucleotides.

The CD25 aptamer-drug conjugate of the present invention may be prepared by methods known in the art, for example, by a general oligonucleotide synthesis method, a method of reacting a nucleic acid aptamer with a drug, *etc.,* but is not limited thereto.

Specifically, the oligonucleotide synthesis method may be, but is not limited to, adding a modified nucleic acid, instead of a base, during synthesis of the nucleic acid aptamer, thereby preparing an aptamer comprising the modified nucleic acid in the aptamer sequence, but is not limited thereto.

The method of reacting a nucleic acid aptamer with a drug may comprise, specifically, an aptamer preparation step of preparing a nucleic acid aptamer having an aptamer nucleotide sequence that specifically binds to CD25; and a drug conjugate preparation step of preparing a drug conjugate by reacting the nucleic acid aptamer with the drug. The drug conjugate preparation step may be, for example, adding the drug and an activator dissolved in dimethyl sulfoxide (DMSO) to the nucleic acid aptamer and reacting the same for several minutes, but is not limited thereto.

A specific reaction scheme for synthesizing the CD25 aptamer-drug conjugate may be exemplified as follows:

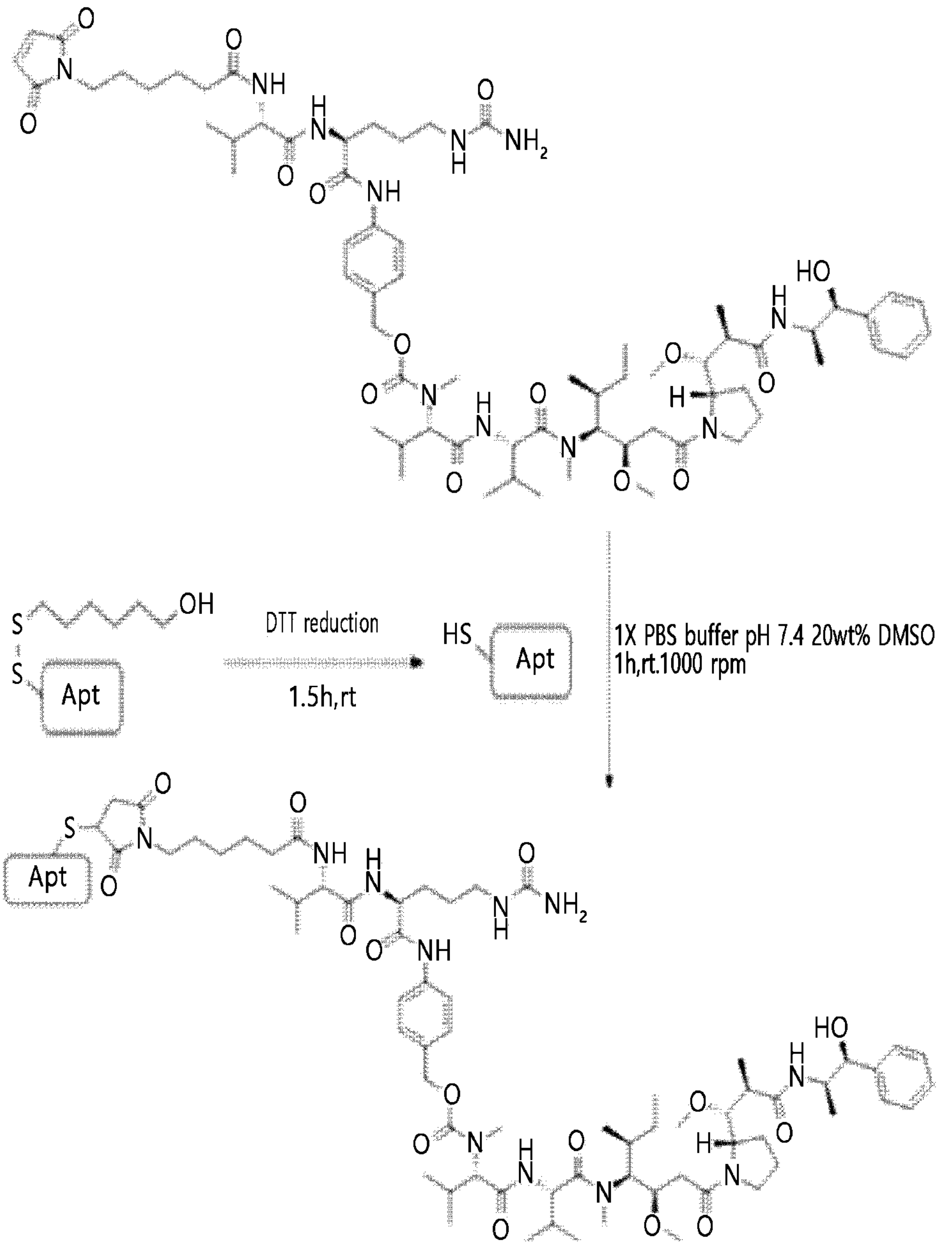


Another specific reaction scheme for synthesizing the drug conjugate may be exemplified as follows:

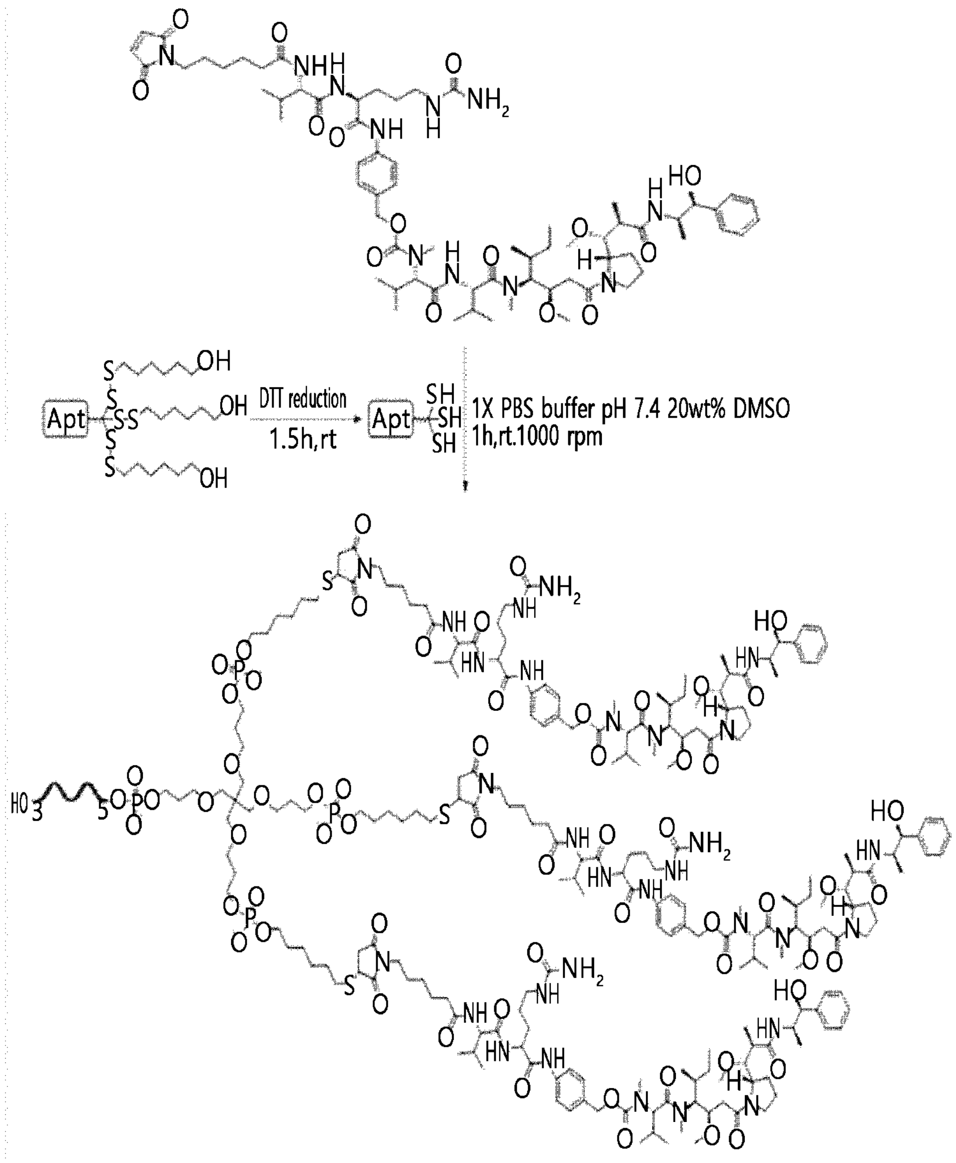


For example, the CD25 aptamer-drug conjugate of the present invention may selectively eliminate Tregs.

In a specific embodiment of the present invention, it was confirmed that CD25-MMAE(1) (DAqR=1) and CD25-MMAE(3) (DAqR=3) which are the CD25 aptamer-drug conjugates of the present invention exhibit no selective toxicity to a CD25-negative cell line, while exhibiting selective toxicity to a CD25-positive Treg-like cell line (FIG. 19). It was also confirmed that they exhibit the selective killing effect only on the CD25-positive Treg-like cell line in a co-culture of CD25-positive Treg-like cell line and CD25-negative cell line (FIG. 20).

A third aspect of the present invention provides a pharmaceutical composition for treating cancer diseases, the pharmaceutical composition comprising the CD25 aptamer of the first aspect or the CD25 aptamer-drug conjugate of the second aspect as an active ingredient.

As used herein, the terms "CD25 aptamer of the first aspect" and "CD25 aptamer-drug conjugate of the second aspect" are as described above.

As used herein, the term "treating" means all of the actions by which symptoms of cancer diseases have taken a turn for the better or been modified favorably by the administration of the composition of the present invention.

As described above, the CD25 aptamer of the present invention may bind to CD25 protein and act as an IL-2 antagonist, specifically, exhibits effects of inhibiting the binding between CD25 and IL-2, inhibiting signal transduction by the binding between CD25 and IL-2, reducing the level of STAT5 phosphorylation by the binding between CD25 and IL-2, or reducing the level of TGF-β by the binding between CD25 and IL-2, and may suppress the activation of CD25-positive T cells, and the CD25 aptamer-drug conjugate may selectively remove Tregs and may exhibit the selective killing effect only on CD25-positive Treg-like cell line, and therefore, the pharmaceutical composition comprising the same as an active ingredient may be useful for the treatment of cancer diseases, which are diseases related thereto. For example, the

pharmaceutical composition of the present invention may be used to treat lung cancer such as non-small cell lung cancer, small cell lung cancer, malignant mesothelioma, *etc.;* mesothelioma; pancreatic cancer such as pancreatic ductal adenocarcinoma, pancreatic neuroendocrine tumor, *etc.;* pharyngeal cancer; laryngeal cancer; esophageal cancer; gastric cancer such as papillary adenocarcinoma, mucinous adenocarcinoma, adenosquamous carcinoma, *etc.;* duodenal cancer; small intestine cancer; colorectal cancer such as colon cancer, rectal cancer, anal cancer, familial colorectal cancer, hereditary nonpolyposis colorectal cancer, gastrointestinal stromal tumor, *etc.;* breast cancer such as invasive ductal carcinoma, non-invasive ductal carcinoma, inflammatory breast cancer, *etc.;* ovarian cancer such as epithelial ovarian carcinoma, extragonadal germ cell tumor, ovarian germ cell tumor, ovarian low-grade tumor, *etc.;* testicular tumor; prostate cancer such as hormone-dependent prostate cancer, hormoneindependent prostate cancer, *etc.;* liver cancer such as hepatocellular carcinoma, primary liver cancer, extrahepatic cholangiocarcinoma, *etc.;* thyroid cancer such as medullary thyroid carcinoma, *etc.;* renal cancer such as renal cell carcinoma, transitional cell carcinoma of renal pelvis and ureter, *etc.;* uterine cancer such as cervical cancer, endometrial cancer, uterine sarcoma, *etc.;* brain tumor such as medulloblastoma, glioma, pineal germinoma, pilocytic astrocytoma, diffuse astrocytoma, degenerative astrocytoma, pituitary adenoma, *etc.;* retinoblastoma; skin cancer such as basal cell carcinoma, malignant melanoma, *etc.;* sarcoma such as rhabdomyosarcoma, leiomyosarcoma, soft tissue sarcoma, *etc.;* malignant bone tumor; and bladder cancer, *etc.,* but is not limited thereto. Furthermore, CD25 expression has been reported for several B- and T-cell-derived subtypes of non-Hodgkin's lymphoma, *i.e*., B-cell chronic lymphocytic leukemia, hairy cell leukemia, small lymphocytic lymphoma/-chronic lymphocytic leukemia, as well as adult T-cell leukemia/lymphoma and anaplastic large cell lymphoma, and thus the pharmaceutical composition may be useful in the treatment of these diseases.

Preferably, the pharmaceutical composition according to the present invention may comprise the CD25 aptamer or CD25 aptamer-drug conjugate as an active ingredient in an amount of 0.0001% by weight to 75% by weight, for example, 0.001% by weight to 50% by weight, 0.0001% by weight to 10% by weight, 0.001% by weight to 10% by weight, or 0.001% by weight to 1% by weight, based on the total weight of the composition.

The composition of the present invention may further comprise a pharmaceutically acceptable carrier, diluent or excipient, and may be used after being formulated into various forms according to common methods according to each purpose for use, such as oral formulations such as powders, granules, tablets, capsules, suspensions, emulsions, syrups, aerosols, *etc.,* and injectable formulation of sterile injection solutions, *etc.* The composition may be orally administered or administered through various routes comprising intravenous, intraperitoneal, subcutaneous, rectal, topical administration, *etc.* Examples of suitable carriers, excipients, or diluents that may be comprised in such compositions may comprise lactose, dextrose, sucrose, sorbitol, mannitol, xylitol, erythritol, maltitol, starch, gum acacia, alginate, gelatin, calcium phosphate, calcium silicate, cellulose, methylcellulose, microcrystalline cellulose, polyvinylpyrrolidone, water, methylhydroxybenzoate, propylhydroxybenzoate, talc, magnesium stearate, mineral oil, *etc.* In addition, the composition of the present invention may further comprise a filler, an anti-coagulant, a lubricant, a wetting agent, a flavoring agent, an emulsifier, a preservative, *etc.*

Solid preparations for oral administration may comprise tablets, pills, powders, granules, capsules, *etc.* Such solid preparations may be formulated by mixing the composition with at least one or more excipients, such as starch, calcium carbonate, sucrose, lactose, gelatin, *etc.* Additionally, lubricants such as magnesium stearate and talc may be used, in addition to simple excipients.

Oral liquid preparations may be exemplified by suspensions, internal solutions, emulsions, syrups, *etc.,* and may comprise various excipients, for example, wetting agents, sweeteners, aromatics, preservatives, *etc.,* in addition to simple diluents commonly used, such as water and liquid paraffin.

Preparations for parenteral administration may comprise sterile aqueous solutions, non-aqueous solvents, suspensions, emulsions, freeze-drying preparations, and suppositories. Propylene glycol, polyethylene glycol, vegetable oils such as olive oil and injectable esters such as ethyl oleate may be used as non-aqueous solvents and suspensions. Witepsol, Macrogol, Tween 61, cacao butter, laurin butter, glycerogelatin, *etc.,* may be used as a base for the suppository. Meanwhile, traditional additives such as solubilizers, isotonic agents, suspending agents, emulsifiers, stabilizers, preservatives, *etc.,* may be comprised in the injectable formulation.

In this regard, the composition of the present invention may be administered in a pharmaceutically effective amount. As used herein, the term "pharmaceutically effective amount" means an amount that is sufficient to treat a disease at a reasonable benefit/risk ratio applicable to medical treatment and does not cause side effects. The effective dose level may be determined according to factors comprising the patient's health condition, the type of disease, severity, activity of the drug, sensitivity to the drug, method of administration, time of administration, route of administration and excretion rate, duration of treatment, drugs used in combination or concurrently and other factors well known in the medical field. The composition of the present invention may be administered as an individual therapeutic agent or in combination with other therapeutic agents, may be administered sequentially or simultaneously with existing therapeutic agents, and may be administered singly or multiple doses. Considering all of the above factors, it is important to administer an amount that may obtain the maximum effect with the minimum amount without side effects, which may be easily determined by those skilled in the art.

For example, as the dosage may increase or decrease depending on the route of administration, severity of disease, sex, body weight, age, *etc.,* the dosage does not limit the scope of the present invention in any way.

Specifically, the effective amount of the compound in the composition of the present invention may vary depending on a patient's age, sex, and body weight. In general, 0.001 mg to 1000 mg, specifically 0.05 mg to 2000 mg, more specifically 0.1 mg to 100 mg per kg of body weight may be administered daily, every other day or divided into 1 to 3 times per day. However, as the dosage may increase or decrease depending on the route of administration, severity of disease, sex, body weight, age, *etc.,* the dosage does not limit the scope of the present invention in any way.

A fourth aspect of the present invention provides a method of treating cancer diseases, the method comprising the step of administering the pharmaceutical composition of the third aspect to a subject in need thereof.

As used herein, the terms "pharmaceutical composition of the third aspect", "cancer diseases", and "treating" are as described above.

As used herein, the term "subject" means any animals, comprising monkeys, cows, horses, sheep, pigs, chickens, turkeys, quails, cats, dogs, mice, rats, rabbits, or guinea pigs, as well as humans who have developed or may develop the cancer diseases. The diseases may be effectively treated by administering the pharmaceutical composition of the present invention to the subject. The pharmaceutical composition of the present invention may be administered in combination with existing therapeutic agents.

As used herein, the term "administering" means providing a predetermined substance to a patient by any suitable method. The administration route of the composition of the present invention may be any general route as long as it may reach a target tissue. The composition of the present invention may be administered by intraperitoneal administration, intravenous administration, intramuscular administration, subcutaneous administration, intradermal administration, oral administration, topical administration, intranasal administration, intrapulmonary administration or intrarectal administration, but is not limited thereto. In addition, the pharmaceutical composition of the present invention may be administered by any device capable of transporting an active substance to a target cell. Preferred administration modes and preparations may be intravenous injections, subcutaneous injections, intradermal injections, intramuscular injections, drop injections, *etc.* Injections may be prepared using aqueous solvents such as physiological saline, Ringer's solution, *etc.,* or non-aqueous solvents such as vegetable oil, higher fatty acid esters (*e.g.*, ethyl oleate, *etc.),* alcohols (*e.g.*, ethanol, benzyl alcohol, propylene glycol, glycerin, *etc.),* and may comprise a pharmaceutical carrier such as stabilizers for preventing deterioration (*e.g.*, ascorbic acid, sodium hydrogensulfite, sodium pyrosulfite, BHA, tocopherol, EDTA, *etc.),* emulsifiers, buffers to control pH, preservatives to inhibit microbial growth (e.g., phenylmercuric nitrate, thimerosal, benzalkonium chloride, phenol, cresol, benzyl alcohol, *etc.).*

The term "therapeutically effective amount" used in combination with the active ingredient in the present invention means an amount of the CD25 aptamer or CD25 aptamer-drug conjugate effective for treating a target disease.

Depending on the type of diseases to be prevented or treated, the pharmaceutical composition of the present invention may further comprise known drugs used for preventing or treating each disease, in addition to the CD25 aptamer or CD25 aptamer-drug conjugate, as an active ingredient. For example, the composition of the present invention may further comprise known drugs, in addition to the CD25 aptamer or CD25 aptamer-drug conjugate, as an active ingredient, when the composition is used for preventing or treating cancer diseases, and may be used in combination with other known therapies for treating these diseases.

## [Mode for Carrying Out the Invention]

Hereinafter, the present invention will be described in more detail with reference to exemplary embodiments. However, these exemplary embodiments are only for illustrating the present invention, and the scope of the present invention is not intended to be limited thereby.

### Example 1: Preparation of CD25 Protein-Specific Aptamers

As CD25 protein-specific aptamers, a total of three types of aptamers binding to CD25 (IL-2R$\alpha$) protein, which corresponds to the alpha subunit of interleukin-2 (IL-2) receptor, were discovered through Systematic evolution of ligands by exponential enrichment (SELEX), which is a general process for discovering aptamers, and prepared as shown in Table 1 below through chemical synthesis of selected sequences.

[Table 1]

| SEQ ID NO. | Name | Sequence (5'→3') | Length |
|---|---|---|---|
| 4 | CD25-22 18-11-02 | AGTTCG**T**G**T**G**T**G**T**CAC**T**C**T**A**T**CG**TT**ACGAGGAA**T**A CGGGCAAAGCGACCA | 50 mer |

(continued)

| SEQ ID NO. | Name | Sequence (5'→3') | Length |
|---|---|---|---|
| 5 | CD25-22 35-15-02 | AGTTCCGG**T**GCAA**T**CGCG**T**GG**T**CAACA**T**CC**T**G**TT**AACC**T**CGGAA**T**GACCA | 50 mer |
| 6 | CD25-22 64-10-02 | AGTTCGAGA**T**CAAG**TT**A**T**AGCG**T**G**T**CCCA**TT**C**T**ATCCAAGACAC**T**GACCA | 50 mer |

The bolded bases above represent a thymine or uracil base substituted with 5-naphthylmethylaminocarbonyl-dU (NapdU).

**Example 2: Confirmation of Binding Potential to CD25 Protein, Cell Binding Potential, and Internalization of CD25 Aptamer**

**2-1. Binding Potential of CD25 Aptamer to IL-2 Receptor Protein**

To confirm the binding potential of the three types of aptamers obtained from Example 1 to CD25 protein, the three types of aptamers were diluted with 1× $SB18_{T0.05}$ buffer (200 mM hydroxyethyl piperazine ethane sulfonic acid (HEPES), 510 mM NaCl, 25 mM $MgCl_2$, 25 mM KCl, 0.05% [v/v] tween-20) to prepare 100 μL of 1 nM aptamer solution. This solution was heated at 95°C, 70°C, 48°C, and 37°C each for 5 minutes, and then cooled at room temperature (RT) for 15 minutes to perform heating and cooling of the aptamers. As recombinant human IL-2 receptor proteins, 100 nM of rhCD25(IL-2Rα), rhCD122(IL-2Rβ), and rhCD132(IL-2Rγ) proteins were serially diluted by 4.64-fold at 7 points using 1× $SB18_{T0.05}$ buffer, and each 30 μL thereof was dispensed into a 96-well plate. 200 μL of 1× $SB18_{T0.05}$ buffer was added to the heated and cooled aptamer, mixed, and 30 μL thereof was added to each well of the 96-well plate, and a binding reaction between the aptamer and the protein was allowed at 37°C for 30 minutes. 5.5 μL of 400 mg/mL zorbax beads were added to each well, and the reaction was allowed at 1400 rpm for 5 minutes in a thermomixer. The reaction product where the reaction was completed was placed on a Durapore filter plate (hydrophilic low protein binding Durapore 0.45 μm filter plate) pre-wetted with 30 μL of 1× $SB18_{T0.05}$ buffer. The buffer was removed by vacuum, and then the plate was then washed three times with 200 μL of 1× $SB18_{T0.05}$ buffer.

20 μL of 8 mM HCl was added to a 1 mL deep-well plate, and the filter plate was placed thereon. 80 μL of 2 mM NaCl was then added to the well, in which the binding reaction between the aptamer and then protein had been allowed. The plate was incubated in a thermomixer at 1000 rpm for 5 minutes to elute the proteinbound aptamers, followed by centrifugation at 1,000 rpm for 1 minute. The eluted aptamer solution and the HCl solution in the deep-well plate were mixed to neutralize the solution. Each 0.04 μL of 100 μM forward primer and reverse primer, 10 μL of AccuPower 2X GreenStar™ qPCR MasterMix (-ROX Dye, Bioneer), 0.1 μL of 1 M $MgCl_2$, and 9.82 μL of the eluted aptamer solution were mixed to a total of 20 μL, and then amplified by qPCR. Sequences of the forward and reverse primers used are shown in Table 2. The PCR conditions were 1 cycle of 96°C for 15 seconds, 55°C for 10 seconds, and 68°C for 30 minutes, and then 40 cycles of 96°C for 15 seconds and 72°C for 1 minute. The Ct values were normalized to the standard, and Kd was calculated using SigmaPlot.

[Table 2]

| SEQ ID NO. | Name | Sequence (5'→3') |
|---|---|---|
| 7 | 2218-11-02-For | GGA GTT CGT GTG TGT CAC TCT ATC GTT |
| 8 | 2218-11-02-Rev | GTC GCT TTG CCC GTA TTC CTC G |
| 9 | 2235-15-02-For | GTT CCG GTG CAA TCG CGT G |
| 10 | 2235-15-02-Rev | GGA GGT CAT TCC GAG GTT AAC AGG A |
| 11 | 2264-10-02-For | GGA GTT CGA GAT CAA GTT ATA GCG TGT CC |
| 12 | 2264-10-02-Rev | GGA GGT CAG TGT CTT GGA TAG AAT GGG |

As a result, as shown in FIGS. 1 to 3, all three types of aptamers exhibited a high binding potential of nM or less to human CD25 (IL-2Rα) protein, which corresponds to the alpha subunit of human IL-2 receptor, and 2264-10-02 (SEQ ID

NO: 6) exhibited the highest binding potential to CD25 protein.

In addition, all three types of aptamers did not exhibit binding potential to human CD122 (IL-2Rβ) and CD132 (IL-2Rγ), which correspond to the beta and gamma subunits of human IL-2 receptor, respectively, confirming their specific binding to CD25 (IL-2Rα).

### 2-2. Cell Binding Potential of CD25 Aptamer

To confirm the cell binding potential of the three types of aptamers of Example 1, Karpas299 cells which are CD25-positive cells or Daudi cells which are CD25-negative cells were counted at 1 × $10^6$ cells/sample and collected in a fluorescence-activated cell sorting (FACS) tube, centrifuged at 1000 rpm for 3 minutes at 4°C, and washed once with 1× phosphate-buffered saline (PBS) buffer. After heating at 95°C for 5 minutes and cooling at room temperature for 15 minutes, the three types of aptamers thus heated and cooled were diluted to a final concentration of 100 nM using ice-cold FACS buffer (1× PBS buffer comprising 5 mM $MgCl_2$), and treated to the cells, followed by incubation at 4°C in the dark for 30 minutes. After centrifugation at 1,000 rpm for 3 minutes at 4°C, the supernatant was discarded, and washing by gentle mixing with 2 mL of 1× PBS buffer was repeated three times. After centrifugation again at 1,000 rpm for 3 minutes at 4°C, the supernatant was discarded, and 1 mL of FACS buffer was added. The cells were transferred to new strainer-cap FACS tubes and analyzed using a flow cytometer (BD FACS Calibur).

As shown in FIG. 4, it was confirmed that all three types of aptamers specifically bound only to the CD25-positive cell line Karaps299. It was also confirmed that the fluorescence intensity was observed highest in the order of 2218-11-02, 2235-15-02, and 2264-10-02, indicating that the 2264-10-02 aptamer has the highest cell binding potential.

### 2-3. Internalization of CD25 Aptamer

To confirm the internalization of the three types of aptamers of Example 1, cover glass was sterilized by flame and prepared on a 6-well plate. The cover glass was treated with a poly-L-lysine (PLL) solution at room temperature for 15 minutes, and then washed three times with sterile distilled water which was filtered with 0.45 μm. The cover glass was dried overnight using a UV lamp in a clean bench to remove moisture, and a 6-well plate equipped with the PLL-coated cover glass was prepared. Karpas299 cells were seeded at 1 × $10^6$ cells/sample on the 6-well plate equipped with the PLL-coated cover glass, and then incubated at room temperature for 10 minutes. After heating at 95°C for 5 minutes and cooling at room temperature for 15 minutes, the three types of aptamers thus heated and cooled were diluted to a final concentration of 50 nM using FACS buffer and treated to cells, which were then incubated in the dark at 4°C for 1 hour. 4 hours later, 1× PBS buffer was added, and after 5 minutes at room temperature, the 1× PBS buffer was removed. This procedure was repeated three times. The remaining solution was removed by suction, followed by washing with 1× PBS buffer. A complete medium (RPMI1640 medium comprising 10% fetal bovine serum (FBS) and 1% penicillin/streptomycin) was added, and the cells were incubated in a $CO_2$ incubator for 4 hours, followed by washing with 1× PBS buffer three times. PBS buffer comprising 4% paraformaldehyde (PFA) was added to each well, and cells were fixed for 15 minutes at 4°C in the dark, followed by washing with 1× PBS buffer three times. A mounting solution comprising 4',6'-diamidino-2-phenylindole (DAPI) was dropped onto a slide glass and covered with a cover glass to perform staining and mounting. The slide was stored in a light-shielding box and refrigerated. Next day, measurement was made using a confocal unit (LSM800).

As a result, as shown in FIG. 4, it was observed that all three types of aptamers bound to the cell surface at 0 hour and internalized into the cytoplasm at 4 hours. These results suggest that the three types of aptamers bind to the CD25 protein on the cell surface and then enter CD25-positive cells through receptormediated endocytosis (RME).

## Example 3: Confirmation of Inhibition of IL-2/IL-2Ra Binding and Consequent Cell Signaling by CD25 Aptamer

### 3-1. Inhibition of IL-2/IL-2Ra Binding by CD25 Aptamer

To confirm whether or not the three types of aptamers of Example 1 inhibit the binding of IL-2/IL-2Rα, 100 μL of 100 ng/mL rhCD25 protein was placed in a 96-well enzyme-linked immunosorbent assay (ELISA) plate, and incubated overnight on a shaker to coat the inside of the wells with rhCD25 protein. After removing the residual solution from the wells, they were washed three times using 200 μL of 1× PBST buffer (PBS buffer comprising 0.1% Tween 20). Each 50 μL of the three types of aptamers, which were diluted with FACS buffer to a final concentration of 100 nM, 50 nM, and 10 nM, were added to each well coated with CD25 protein and allowed to react at 400 rpm for 30 minutes at room temperature using a thermomixer. After removing the residual solution, 50 μL of 50 ng/mL biotin-conjugated IL-2 protein was added to each well and allowed to react at room temperature for 1 hour and 30 minutes at 400 rpm using the thermomixer. After removing the residual solution, the wells were washed three times with 200 μL of 1× PBST buffer. 100 μL of horseradish peroxidase (HRP)-streptavidin solution was added to each well and allowed to react at room temperature for 30 minutes at 400 rpm

using the thermomixer. After removing the residual solution, the wells were washed three times with 200 μL of 1× PBST buffer. 50 μL of 3,3',5,5'-tetramethylbenzidine (TMB) substrate solution was added to each well and allowed to react at room temperature for 1 hour and 30 minutes at 400 rpm using the thermomixer. 50 μL of a stop solution was added to each well, and OD values were measured at 450 nm using a plate reader. From the measured values, a percentage to the control group was calculated, and the results are shown in FIG. 5.

As a result, as shown in FIG. 5, the competitive ELISA confirmed that all three aptamers bind to the CD25 protein at the same binding site as the IL-2 protein.

**3-2. Inhibition of IL-2/IL-2Ra Cell Signaling by CD25 Aptamers**

To determine changes in the STAT5 phosphorylation (pSTAT5) levels in the IL-2/IL-2Rα cell signaling pathway by treatment with 2264-10-02 of Example 1, Karpas299 cells were seeded at 1 × $10^6$ cells/sample in a 6-well plate and incubated overnight to induce serum starvation. The aptamer 2264-10-02, which was diluted with FACS buffer to final concentrations of 100 nM, 50 nM, and 10 nM, was treated to cells. 30 minutes later, IL-2 protein at a final concentration of 10 U/mL was treated to the cells, and 15 minutes later, Western blotting was performed.

To perform Western blotting, cells treated as described above were homogenized with 1× RIPA buffer (50 mmol/L Tris comprising 1% NP-40, 0.5% sodium deoxycholate (DOC), 0.1% SDS, and 150 mmol/L NaCl, pH 8.0,; Sigma-Aldrich) and 1× protease inhibitor. Next, samples were mixed with 5× loading buffer and boiled at 100°C for 10 minutes. 10 μg of extracted protein was separated on a 12% polyacrylamide gel by SDS-PAGE, and transferred to a nitrocellulose membrane (10600001, GE Healthcare). Protein concentrations were quantified using a Quick Start Bradford protein assay (Bio-Rad). The membrane was blocked with 5% bovine serum albumin (BSA) and incubated with primary antibodies overnight at 4°C. The membrane was washed three times with Tris-buffered saline (TBS) comprising 0.1% Triton-X100, and then incubated with secondary antibodies for 2 hours at room temperature. Each protein band was visualized using an ECL kit (DG-WF200; Dogen) and quantified using ImageJ (NIH) software. The results are shown in FIG. 6. The used antibodies were antipSTAT5 and anti-β-actin, and β-actin was used as a loading control.

As a result, as shown in FIG. 6, it was confirmed that when treated with 2264-10-02, IL-2-stimulated pSTAT5 levels decreased. As the concentration of the treated aptamer increased to 25 nM, 50 nM, and 100 nM, the IL-2-stimulated pSTAT5 expression levels decreased by 85%, 65%, and 42%, respectively, resulting in an approximate $IC_{50}$ value of 73.96 nM.

As confirmed above, the aptamer of the present invention blocks the binding between CD25 and IL-2 to effectively inhibit the subsequent signaling, thereby reducing pSTAT5 levels, and thus it has the effect of suppressing the activity of CD25-positive cells.

**Example 4: Optimization of Length of CD25 aptamer**

To optimize the length of aptamer 2264-10-02, which showed the highest cell binding potential to CD25-positive cells in Example 2-2, its secondary structure was simulated through RNAstructure and its tertiary structure was simulated through RNAComposer. A total of seven aptamers with minimum lengths (46 mer, 43 mer, 40 mer, 39 mer, 33 mer, 30 mer, and 27 mer) that maintained the stem and loop regions were additionally prepared, as shown in Table 2 (FIG. 7).

[Table 3]

| SEQ ID NO. | Name | Sequence (5'→3') | Length |
|---|---|---|---|
| 6 | CD25-22 | AGTTCGAGATCAAGTTATAGCGTGTCCCATTC | 50 mer |
| | 64-10-02 | TATCCAAGACACTGACCA | |
| 13 | CD25-22 64-10-05 | GCGTGTCCCATTCTATCCAAGACACTG | 27 mer |
| 14 | CD25-22 64-10-06 | GAGATCAAGTTATAGCGTGTCCCATTCTATCC AAGACACT | 40 mer |
| 15 | CD25-22 64-10-07 | ATCAAGTTATAGCGTGTCCCATTCTATCCAAGA CACTG | 38 mer |
| 16 | CD25-22 64-10-08 | GTTATAGCGTGTCCCATTCTATCCAAGACACT G | 33 mer |
| 17 | CD25-22 64-10-09 | ATAGCGTGTCCCATTCTATCCAAGACACTG | 30 mer |

(continued)

| SEQ ID NO. | Name | Sequence (5'→3') | Length |
|---|---|---|---|
| 18 | CD25-22 64-10-10 | TTCGAGA**T**CAAG**TT**A**T**AGCG**T**G**T**CCCA**TT**C**T**A**T** CCAAGACAC**T**GAC | 46 mer |
| 19 | CD25-22 64-10-11 | TTCGAGA**T**CAAG**TT**A**T**AGCG**T**G**T**CCCA**TT**C**T**A**T** CCAAGACAC**T** | 43 mer |

The bolded bases represent thymine or uracil bases substituted with 5-naphthylmethylaminocarbonyl-dU (NapdU).

**Example 5:** : **Confirmation of CD25 Protein-Binding Potential, Cell Binding Potential, and Internalization of Length-Optimized CD25 Aptamer**

**5-1. Binding Potential of Length-Optimized CD25 Aptamer to CD25 protein**

To confirm the binding potential of the seven types of aptamers of Example 4 to CD25 protein, a binding reaction with rhCD25 (IL-2Rα) protein was performed using the method of Example 2-1, and the Ct value was normalized to the standard, and the Kd was calculated using SigmaPlot, and the results are shown in FIG. 8. Sequences of forward and reverse primers used are as follows (Table 4).

[Table 4]

| SEQ ID NO. | Name | Sequence (5'→3') |
|---|---|---|
| 20 | 2264-10, 27 | GGA CGA GCA GCG TGT CCC ATT C |
| | ~38mer For | |
| 21 | 2264-10, 27 ~38mer Rev | GGA CGA GCA AGT GTC TTG GAT AG |
| 22 | 2264-10, 40 ~46mer For | GGA CGA GCA GAG ATC AAG TTA TAG CG |
| 23 | 2264-10, 40 ~46mer Rev | GGA CGA GCA AGT GTC TTG GAT AGA ATG |

As a result, as shown in FIG. 8, among the seven types of aptamers, CD25-2264-10-10 (SEQ ID NO: 18) and CD25-2264-10-11 (SEQ ID NO: 19), each having lengths of 46 mer and 43 mer, were confirmed to have the binding potential to human CD25 protein.

Accordingly, the 5'-terminal helical structure in the tertiary structure of the aptamer is important for binding to CD25 protein.

**5-2. Cell Binding Potential of Length-Optimized CD25 Aptamer**

To confirm the cell binding potentials of 2264-10-02 of Example 1 and CD25-2264-10-10 and CD25-2264-10-11 of Example 4, which were length-optimized to have the length of 46 mer and 43 mer, respectively, binding reactions for Karpas299 cells or Daudi cells were performed using the method of Example 2-2, and measurements were made using a flow cytometer (Beckman CytoFlex). The results are shown in FIG. 9.

As a result, as shown in FIG. 9, it was confirmed that the aptamer 2264-10-02 and its length-optimized aptamers CD25-2264-10-10 and CD25-2264-10-11 all exhibited the binding potential to CD25-positive cells, as compared to the negative control aptamer (NC aptamer) and CD25-negative cells.

**5-3. Internalization of Length-Optimized CD25 Aptamer**

To confirm the internalization of CD25-2264-10-11 with the length of 43 mer, which was confirmed to have the excellent cell binding potential as in Example 5-2, an internalization reaction for Karpas299 cells or Daudi cells was performed using the method of Example 2-3, and measurements were made using a confocal unit (LSM800). As a negative control aptamer (NC aptamer), NC aptamer, in which its sequence was the same as the 2264-10-11 aptamer, but the thymine base was not substituted with NapdU, was used (Table 5).

[Table 5]

| SEQ ID NO. | Name | Sequence (5'→3') |
|---|---|---|
| 24 | NC aptamer | TTCGAGATCAAGTTATAGCGTGTCCCATTCTATCCAA GACACT |

As a result, as shown in FIG. 10, it was confirmed that the length-optimized CD25-2264-10-11 exhibited selective internalization into CD25-positive cells.

### 5-4. Internalization Rate of Length-Optimized CD25 Aptamer

To determine the internalization rate of CD25-2264-10-11 of Example 4, Karpas299 cells were counted at $1 \times 10^6$ cells/sample, collected in a FACS tube, centrifuged at 1000 rpm for 3 minutes at 4°C, and washed once with 1× PBS buffer. The cells were resuspended in ice-cold 1× PBS buffer, and pre-chilling was performed at 4°C for 30 minutes. Then, centrifugation was performed at 1000 rpm for 3 minutes at 4°C and the supernatant was discarded. After heating at 95°C for 5 minutes and cooling at room temperature for 15 minutes, the heated and cooled aptamer was diluted to a final concentration of 100 nM using ice-cold FACS buffer and treated to the cells. The cells were incubated in the dark at 4°C for 30 minutes. Centrifugation was performed at 1000 rpm for 3 minutes at 4°C, the supernatant was discarded, and 2 mL of 1× PBS buffer was added and mixed gently to wash. Complete medium was added and incubated in a $CO_2$ incubator at 37°C for 15 minutes, 30 minutes, 60 minutes, 120 minutes, 240 minutes, and 300 minutes. Centrifugation was performed at 1000 rpm for 3 minutes at 4°C, the supernatant was discarded, and 2 mL of 1× PBS buffer was added and mixed gently to wash. The FACS tubes were divided into two. PBS buffer was placed in one tube for cell binding conditions, and a buffer comprising 0.1 M glycine at pH 10.0 and 4 g/L NaCl was placed in the other tube for internalization conditions, followed by incubation at 37°C for 15 minutes. Then, measurements were made using a flow cytometer (Beckman CytoFlex). The internalization rate at each time point was calculated using median fluorescence intensity (MFI) values through the following equation:

$$\text{Internalization rate (\%)} = 100 \times \frac{\text{(Internalized MFI) - (Unstained Control MFI)}}{\text{(Total Binding MFI) - (Unstained Control MFI)}}$$

As a result, as shown in FIG. 11, the internalization rate of aptamer CD25-2264-10-11 was 92% at 30 minutes of incubation, confirming that the internalization rate was fast (Half-time: 9.9 min (95% CI - 8.2-12.6 min)).

## Example 6: Confirmation of Inhibition of IL-2/IL-2Ra Binding and Consequent Cell Signaling by Length-Optimized CD25 Aptamer

### 6-1. Inhibition of IL-2/IL-2Ra Binding by Length-Optimized CD25 Aptamer

To confirm whether or not CD25-2264-10-11 of Example 4 inhibits the binding of IL-2/IL-2Rα, a 96-well ELISA plate was coated with 200 ng/mL rhCD25 protein using the method of Example 3-1. 50 μL of aptamer, serially diluted 4.64-fold from 1 nM to 100 nM, was added to each well coated with CD25 protein and allowed to react at 400 rpm for 30 minutes at room temperature using a thermomixer. After removing the residual solution, 50 μL of 400 ng/mL biotin-conjugated IL-2 protein was added to each well and allowed to react at 400 rpm for 1 hour and 30 minutes at room temperature using a thermomixer. The subsequent process was performed in the same manner as in Example 3-1, and OD values were measured at 450 nm using a plate reader. The percentages to the control group were calculated from the above measurement values, and the results are shown in FIG. 12.

As a result, as shown in FIG. 12, the aptamer CD25-2264-10-11 was confirmed to act as an antagonist in the competitive ELISA.

### 6-2. Inhibition of IL-2/IL-2Ra Cell Signaling by Length-Optimized CD25 Aptamer

To examine the changes mRNA levels of transforming growth factor-β (TGF-β) in the IL-2/IL-2Rα cell signaling pathway by treatment with CD25-2264-10-11 of Example 4, Karpas299 cells were seeded at $1 \times 10^6$ cells/sample in a 6-well plate and incubated overnight to induce serum starvation. The cells were treated with the CD25-2264-10-11 aptamer, which was diluted with FACS buffer to final concentrations of 200 nM, 100 nM, and 50 nM. 30 minutes later, the cells were treated with IL-2 protein at a final concentration of 10 U/mL. 24 hours later, tRNA was recovered from each sample using an

RNA extraction kit (Qiagen). cDNAwas synthesized from 1 μg of tRNA using a cDNA synthesis kit (Takara), and used as a template to perform qPCR. To this end, 0.4 μL of 10 μM forward and reverse primers, 10 μL of AccuPower 2× GreenStar™ qPCR MasterMix (-ROX Dye, Bioneer), 2 μL of cDNA template, and 7.2 μL d.w. were mixed to prepare a total volume of 20 μL, and amplified using qPCR. The PCR conditions were 95°C for 2 minutes, followed by 40 cycles of 95°C for 15 seconds, 65°C for 30 seconds, and 72°C for 30 seconds. Sequences of the primers used are shown in Table 6 below. Furthermore, the normalized TGF-β mRNA expression levels were calculated and shown in FIG. 13.

[Table 6]

| SEQ ID NO. | Name | Sequence (5'→3') |
|---|---|---|
| 25 | TGF-β-For | GGC CAG ATC CTG TCC AAG C |
| 26 | TGF-β-Rev | GTG GGT TTC CAC CAT TAG CAC |

As a result, as shown in FIG. 13, treatment with the CD25-2264-10-11 aptamer significantly reduced the increase in the mRNA expression of TGF-β by IL-2 stimulation. Specifically, as the aptamer concentration increased to 50 nM, 100 nM, and 200 nM, the mRNA expression level of TGF-β by IL-2 stimulation decreased by 23%, 5%, and 4%, respectively, resulting in an approximate $IC_{50}$ value of 29.07 nM.

As confirmed above, this indicates that the aptamer of the present invention blocks the binding between CD25 and IL-2 to effectively inhibit the subsequent signaling, thereby reducing TGF-β levels, and thus it may exhibit the effect of suppressing the activity of CD25-positive regulatory Tregs.

**Example 7: Chemical Optimization of Length-Optimized CD25 Aptamer**

To chemically optimize the aptamer CD25-2264-10-11 prepared in Example 4, as shown in Table 3 below, the aptamer was modified by additionally introducing a uracil (U) base ($U_{Me}$) in which the -OH group at the C2 position at the 5' end of the aptamer sequence was substituted with methoxy, and a guanine (G) base ($G_{Me}$) in which methoxy was substituted at the 3' end of the aptamer sequence in the same way (FIG. 14). The chemically optimized CD25 aptamer sequence, along with its basic sequence, is shown in Table 7 below.

[Table 7]

| SEQ ID NO. | Name | Sequence (5'→3') |
|---|---|---|
| 19 | CD25-22 64-10-11 | TTCGAGA**T**CAAG**TT**A**T**AGCG**T**G**T**CCCA**TT**C**T**A**T**CC AAGACAC**T** |
| 27 | CD25-combi | **U**$_{Me}$TCGAGA**T**CAAG**TT**A**T**AGCG**T**G**T**CCCA**TT**C**T**A**T** CCAAGACAC**TG**$_{Me}$ |

The bolded bases above represent a thymine or uracil base substituted with 5-naphthylmethylaminocarbonyl-dU (NapdU).

**Example 8: Confirmation of Plasma Stability of Chemically-Optimized CD25 Aptamer**

The plasma stability of the chemically optimized CD25 combi aptamer which was prepared in Example 7 was examined.

In detail, 45 μL of 100% mouse plasma was treated with 10 μL of 10 μM CD25-2264-10-11 (SEQ ID NO: 19) or 10 μL of CD25_combi aptamer (SEQ ID NO: 27) and incubated at 37°C for 0 hour, 4 hours, 24 hours, 48 hours, and 72 hours. After incubation, 5 μL of 10 μM control aptamer (a random sequence with a 74-mer length) was treated, followed by dilution with 165 μL of distilled water. An equal volume of a mixed solvent of phenol: chloroform: isoamyl alcohol = 25:24:1 [v/v] was added to the sample and mixed for 20 seconds using a vortex mixer. The mixture was centrifuged at 16,000× g for 10 minutes at room temperature, and the supernatant was transferred to a new tube. Five volumes of sample buffer were added to the supernatant and heated at 95°C for 5 minutes. The sample was added to Urea PAGE and electrophoresed at 220 V for 25 minutes. Then, the gel was stained with SYBR Gold and the gel image was analyzed using a Gel Doc™ EZ system infrared imaging system. The band intensities of the control aptamer and the CD25-2264-10-11 and CD25_combi aptamers were quantified using Image J, and the half-lives were calculated using GraphPad Prism software, and shown in FIG. 15.

As a result, as shown in FIG. 15, the half-life of the chemically-optimized CD25_combi aptamer was 21.49 hours in mouse plasma, 32.48 hours in rat plasma, and 68.27 hours in human plasma, which significantly increased from 20-fold to 8.8-fold, as compared to 7.8 hours of the control aptamer, confirming that the plasma stability was significantly improved by the chemical optimization.

## Example 9: Confirmation of Cell Internalization of Chemically-Optimized CD25 Aptamer via Endosomal-Lysosomal Pathway

To confirm the internalization of the chemically-optimized CD25 combi aptamer prepared in Example 7, Karpas299 cells were counted at $1 \times 10^6$ cells/sample onto poly-L-lysine-coated coverslips. The cells were treated with 100 nM of the chemically-optimized CD25 combi aptamer labeled with the pH-dependent reagent pHrodo RED (Invitrogen), and incubated at 4°C for 30 minutes in the dark, followed by additional incubations at 37°C for 30 minutes, 180 minutes, and 300 minutes. The cells were washed three times by gentle mixing with 1× PBS buffer. The cells were then fixed with 4% PFA/PBS for 15 minutes. The cells were washed three times by gentle mixing with 1× PBS buffer, and then observed under a fluorescence microscope. The results are shown in FIG. 16.

As a result, as shown in FIG. 16, it was confirmed that the chemically-optimized CD25 combi aptamer was internalized via the endosome-lysosome pathway.

## Example 10: Confirmation of Target-Specific Cell Killing by Chemically-Optimized CD25 Combi Aptamer-Drug Conjugate (CD25 Combi ApDC)

### 10-1. Preparation of CD25 combi ApDC (drug to aptamer ratio=1, DApR=1)

For a conjugation reaction of the CD25 combi aptamer of Example 7 with maleimide of MMAE (microtubule inhibitor)-VC (valine-citrulline linker), a CD25 aptamer with a disulfide bond was synthesized, and an SH-CD25 aptamer was then obtained through a reduction reaction using DTT. The SH-CD25 aptamer with a yield of at least 80% or more was recovered by UV quantification. An excess of 20 equivalents of VcMMAE was added to perform conjugation under pH 7.4 buffer conditions. To confirm the synthesis of the CD25 combi aptamer conjugate, purification was performed by HPLC and then an electrophoresis experiment was performed using a urea gel. The final conjugate material was identified by comparing the expected and measured molecular weights through ESI-MS measurement. The synthesis method and the structure of the conjugate material finally synthesized are schematically illustrated in FIG. 17.

### 10-2. Preparation of CD25 combi ApDC (drug to aptamer ratio=3, DApR=3)

For conjugation of the CD25 combi aptamer of Example 7 with 3 molecules of MMAE (microtubule inhibitor)-VC (valine-citrulline linker), a branched disulfide CD25 aptamer was synthesized, and a branched SH-CD25 aptamer was then obtained through a DTT reduction reaction. At least 80% or more of the branched aptamer was recovered by UV quantification. An excess of 25 equivalents of VcMMAE was added to the recovered aptamer to perform conjugation under pH 7.4 buffer conditions. To confirm the synthesis of the branched CD25 combi aptamer conjugate, purification was performed by HPLC and then an electrophoresis experiment was performed using a urea gel. The final conjugate material was identified by comparing the expected and measured molecular weights through ESI-MS measurement. The synthesis method and the structure of the conjugate material finally synthesized are schematically illustrated in FIG. 18. The above reaction was confirmed by HPLC while varying the ratio of drug to SH-CD25 aptamer, and the reaction was considered to complete at the minimum equivalent of 25 equivalents, which is the point where the peak of the reduced SH-CD25 disappeared, and the reaction was allowed at the corresponding ratio.

### 10-3. Confirmation of Specific Killing of CD25-Expressing Cells by CD25 Combi ApDCs

To confirm whether or not the two CD25 combi aptamer conjugates prepared in Examples 10-1 and 10-2 induce specific death of CD25-expressing cells, Karpas299 cells which are CD25-positive cells, or Daudi cells which are CD25-negative cells were seeded in a 96-well plate at a density of 10,000 cells/well, 20,000 cells/well, respectively. Each of the two CD25 combi aptamer conjugates prepared in Examples 10-1 and 10-2 and free MMAE were serially diluted 3.16-fold, starting from 1 μM, 11 times, and treated to each well. The treated cells were incubated for 72 hours, and 2 hours after treatment with a CCK-8 (Dojindo) reagent, the absorbance at 450 nm was measured. Cell viability was calculated from the results, and shown in FIG. 19.

As a result, as shown in FIG. 19, both types of CD25 combi aptamer conjugates were confirmed to induce CD25-specific cell death.

**10-4. Confirmation of Specific Killing of Treg-Like Cells by CD25 combi ApDCs under Coculture Conditions Mimicking Tumor Environment**

To confirm whether or not the two CD25 combi aptamer conjugates prepared in Examples 10-1 and 10-2 induce Treg cell-specific death, $10^6$ cells of Karpas299 cells, which are Treg-like cells, or HuT78 cells, which are Teff-like cells, were seeded on plates, respectively. The two CD25 combi aptamer conjugates, CD25-MMAE (1) and CD25-MMAE (3), were diluted to final concentrations of 40 nM and 10 nM, respectively, and treated to the cell lines, followed by incubation for 24 hours, 48 hours, and 72 hours. The treated cell lines were centrifuged at 1000 rpm for 3 minutes at 4°C, washed with 1× PBS, placed in a 3% BSA/PBS solution, and treated with 0.5 μg of FITC-CD25 (Invitrogen) and 2 μg of AF647-CD4 (R&D systems) for each cell line, and left for 1 hour at 4°C. Each cell line was centrifuged at 1000 rpm for 3 minutes at 4°C, washed with 1× PBS, and then 3% BSA/PBS PI solution was added, and transferred to a strainer cap tube. The ratio of Karpas299 ($CD25^{high}CD4^{high}$) and HuT78 ($CD25^{low}CD4^{low}$) among PI negative (living cells) was examined.

As a result, as shown in FIG. 20, it was confirmed that a higher rate of cell death was induced in the Karpas299 having high CD25 expression, as compared to the HuT78 cell line having low CD25 expression. It was also confirmed that as the ratio of the drug conjugated to the aptamer was higher, the cell death was induced at the faster and higher rate.

As confirmed above, the CD25 aptamer-drug conjugate of the present invention was confirmed to selectively induce the death of cells with high CD25 expression.

The results of the exemplary embodiments suggest that the CD25 aptamer of the present invention specifically binds to the CD25 protein, thereby inhibiting the activation of CD25-positive T cells by binding of IL-2 to the CD25 protein, and the drug conjugate thereof has a shorter half-life than antibodies and thus does not cause side effects, thereby being applied as cancer immunotherapy for cancer treatment.

Based on the above description, it will be understood by those skilled in the art that the present invention may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

## Claims

1. A CD25 aptamer specifically binding to CD25 protein.

2. The CD25 aptamer of claim 1, wherein the aptamer comprises one nucleotide sequence selected from the group consisting of the following General Formulae 1 to 3 or a polynucleotide sequence having 80% or more homology or identity thereto:

   [General Formula 1] TTCGAGATCAAGTTATAGCGTGTCCCATTCTATCCAAGACACT (SEQ ID NO: 1)

   [General Formula 2] GTGTGTGTCACTCTATCGTTACGAGGAATACGGGCAAAGC (SEQ ID NO: 2)

   [General Formula 3] CGGTGCAATCGCGTGGTCAACATCCTGTTAACCTCGGAAT (SEQ ID NO: 3).

3. The CD25 aptamer of claim 1, wherein the aptamer further comprises 1 nucleotide to 30 nucleotides at any one or more of 5'- and 3'-ends thereof.

4. The CD25 aptamer of claim 3, wherein the aptamer comprises 41 nucleotides to 103 nucleotides.

5. The CD25 aptamer of claim 1, wherein the aptamer comprises any one or more modifications selected from the following modifications in one or more nucleotides that constitute its polynucleotide sequence:

   i) substitution of the C5 position of one or more T bases in the nucleotides with any one or more selected from the group consisting of a benzyl group, a naphthyl group, a 2-naphthyl group, a pyrrolebenzyl group, and tryptophan;
   ii) substitution of the -OH group at the C2 position in one or more nucleotides with any one or more selected from

the group consisting of deoxy, methoxy, amino, and fluoro (F); and
iii) substitution of one or more T bases in the nucleotides with $U_{Me}$.

**6.** The CD25 aptamer of claim 5, wherein the aptamer comprises modifications of any one or more nucleotides selected from the group consisting of nucleotides corresponding to positions 8, 13, 14, 16, 21, 23, 28, 29, 31, 33, and 43 of the nucleotide sequence of General Formula 1.

**7.** The CD25 aptamer of claim 6, wherein the aptamer comprises any one or more polynucleotide sequences selected from the group consisting of polynucleotide sequences of SEQ ID NOS: 6, 18, 19, and 27.

**8.** The CD25 aptamer of claim 5, wherein the aptamer comprises modifications of any one or more nucleotides selected from the group consisting of nucleotides corresponding to positions 2, 4, 6, 8, 12, 14, 16, 19, 20, and 29 of the nucleotide sequence of General Formula 2.

**9.** The CD25 aptamer of claim 8, wherein the aptamer comprises a polynucleotide sequence of SEQ ID NO: 4.

**10.** The CD25 aptamer of claim 5, wherein the aptamer comprises modifications of any one or more nucleotides selected from the group consisting of nucleotides corresponding to positions 4, 9, 14, 17, 23, 26, 28, 29, 34, and 40 of the nucleotide sequence of General Formula 3.

**11.** The CD25 aptamer of claim 10, wherein the aptamer comprises a polynucleotide sequence of SEQ ID NO: 5.

**12.** The CD25 aptamer of claim 1, wherein the aptamer acts as an IL-2 antagonist by binding to CD25 protein.

**13.** The CD25 aptamer of claim 1, wherein the aptamer suppresses activation of CD25-positive T cells.

**14.** A CD25 aptamer-drug conjugate (CD25 ApDC) comprising the CD25 aptamer of claim 1, and one or more drugs conjugated to the CD25 aptamer.

**15.** The CD25 aptamer-drug conjugate of claim 14, wherein the drug is an anti-tumor agent.

**16.** The CD25 aptamer-drug conjugate of claim 14, wherein the drug is selected from the group consisting of monomethyl auristatin E (MMAE), monomethyl auristatin F (MMAF), cytarabine, gemcitabine, carboplatin, cisplatin, crizotinib, cyclophosphamide, docetaxel, doxorubicin, erlotinib, etoposide, fluorouracil, imatinib mesylate, irinotecan, methotrexate, paclitaxel, sorafenib, sunitinib, topotecan, trabectedin, vincristine, vinblastine, maytansine, DM1, DM4, calicheamicin and derivatives thereof, doxorubicin, duocarmycin and derivatives thereof, pyrrolobenzodiazepine (PBD), SN-38, $\alpha$-amanitine, and tubulysin analogs.

**17.** The CD25 aptamer-drug conjugate of claim 14, wherein the drug is conjugated to one end of the CD25 aptamer via a chemical linker, conjugated onto the aptamer through interaction between the CD25 aptamer and the drug, or substituted onto the nucleotides constituting the CD25 aptamer.

**18.** The CD25 aptamer-drug conjugate of claim 14, wherein the drug is conjugated to any one of both ends of the CD25 aptamer or onto the nucleotides constituting the aptamer via any one or more chemical bonds of covalent, ionic, metallic, van der Waals, and hydrogen bonds, or inserted between two or more nucleotides constituting the aptamer.

**19.** The CD25 aptamer-drug conjugate of claim 17, wherein the chemical linker comprises a combination of a first linker selected from the group consisting of 5'-thiol-modifier C6, thiol-modifier C6 S-S, dithiol serinol, PC amino-modifier, 5'-amino-modifier C3, 5'-amino-modifier C6, 5'-amino-modifier C12, 5'-amino-modifier TEG, amino-modifier TEG, amino-modifier C2 dT, amino-modifier C6 dT, S-Bz-thiol-modifier C6-dT, phosphodiester bond and nucleotides, and a second linker selected from the group consisting of maleimidocaproyl-valine-citrulline-p-aminobenzoyloxycarbonyl (MC-Val-Cit-PAB), hydrazone, peptide, disulfide, thioether, valine-citrulline, N-maleimidomethyl cyclohexane-1-carboxylate (MCC), maleimidocaproyl, mercaptoacetamidocaproyl, N-succinimidyl 4-(2-pyridyldithio)pentanoate (SPP), succinimidyl 4-(N-maleimidomethyl)cyclohexane-1-carboxylate (SMCC), N-succinimidyl 4-(2-pyridylthio)pentanoate (SPDB), phosphodiester bond and nucleotides.

**20.** A pharmaceutical composition for treating cancer diseases, the pharmaceutical composition comprising the CD25 aptamer of any one of claims 1 to 13 or the CD25 aptamer-drug conjugate of any one of claims 13 to 18 as an active

ingredient.

**21.** The pharmaceutical composition of claim 20, wherein the cancer diseases are selected from the group consisting of lung cancer, mesothelioma, pancreatic cancer, pharyngeal cancer, laryngeal cancer, esophageal cancer, gastric cancer, duodenal cancer, small intestine cancer, colorectal cancer, breast cancer, ovarian cancer, testicular tumor, prostate cancer, liver cancer, thyroid cancer, renal cancer, uterine cancer, brain tumor, retinoblastoma, skin cancer, sarcoma, malignant bone tumor, bladder cancer, B-cell chronic lymphocytic leukemia, hairy cell leukemia, small lymphocytic lymphoma/chronic lymphocytic leukemia, adult T-cell leukemia/lymphoma, and anaplastic large cell lymphoma.

[FIG. 1]

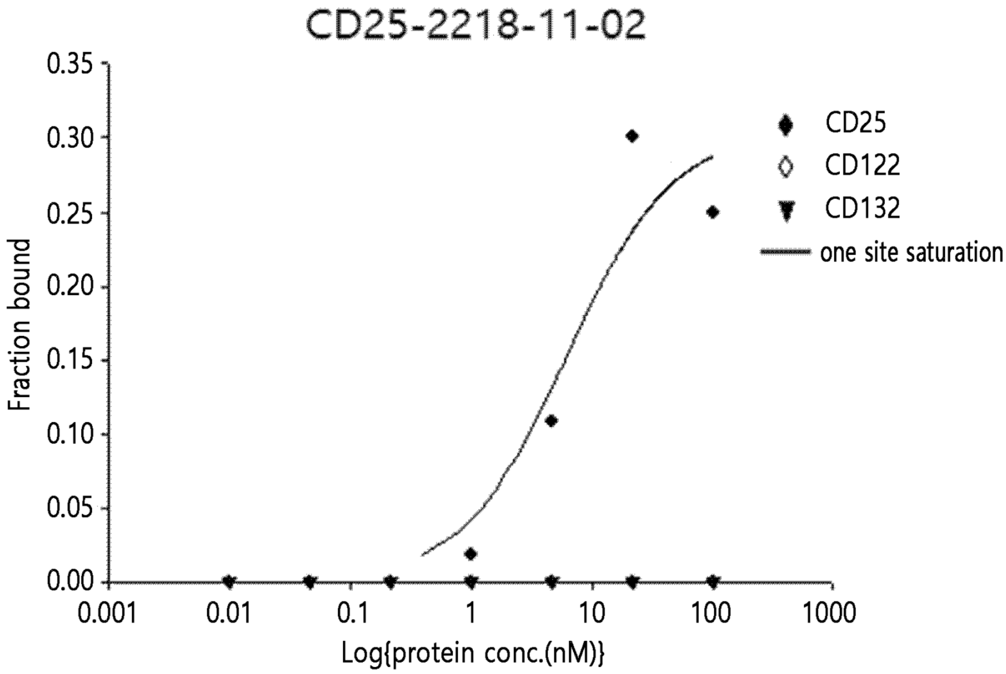


| | CD25 | CD122 | CD132 |
|---|---|---|---|
| Kd(nM) | 6.20 | - | - |
| Bmax | 0.31 | - | - |

[FIG. 2]

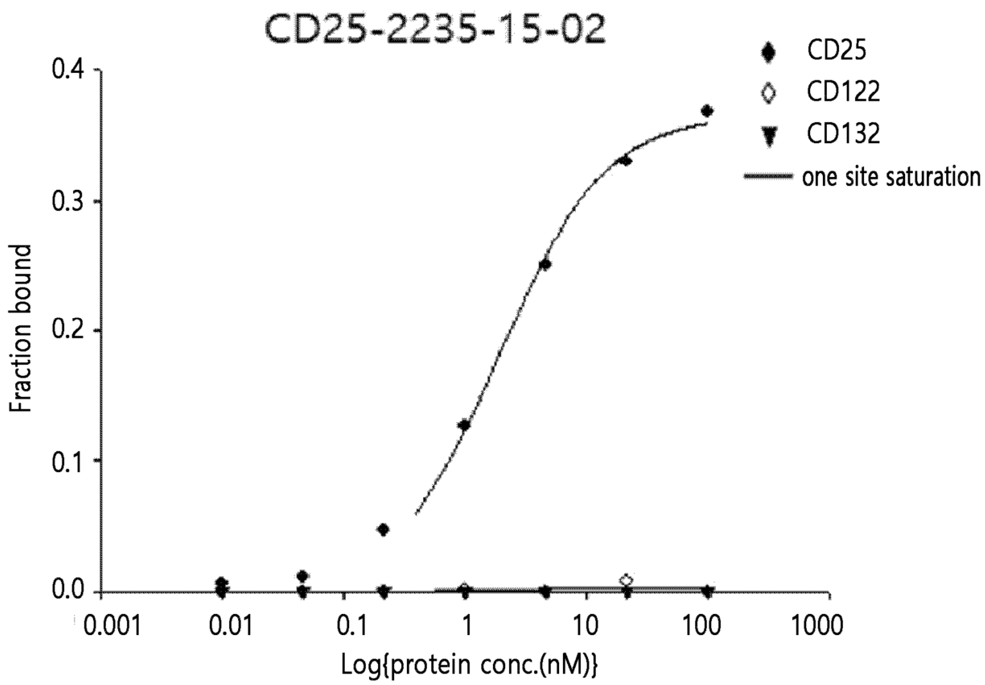


| | CD25 | CD122 | CD132 |
|---|---|---|---|
| Kd(nM) | 6.44 | - | - |
| Bmax | 0.30 | - | - |

[FIG. 3]

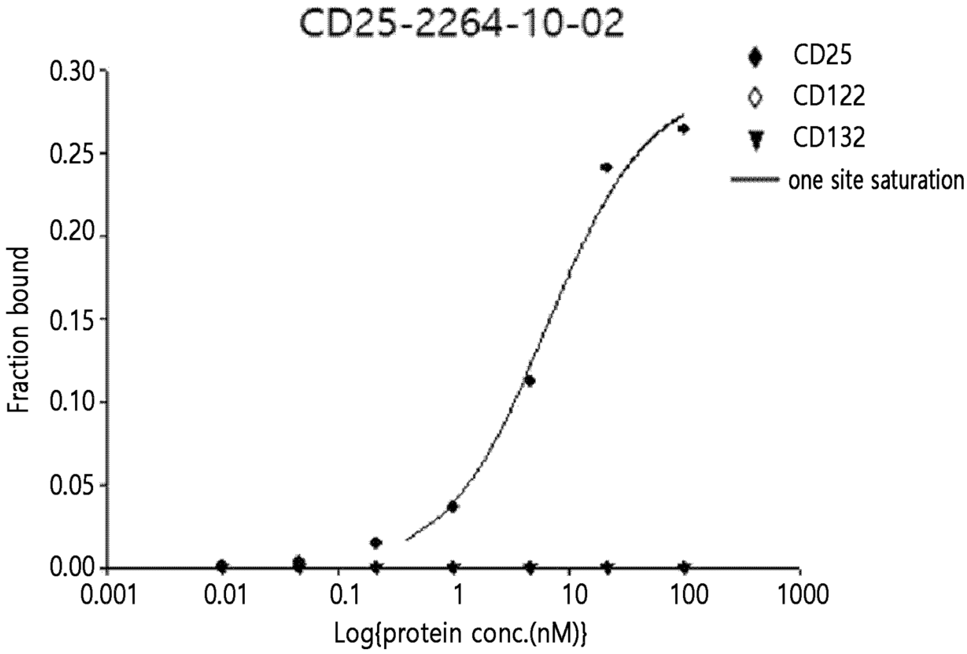


| | CD25 | CD122 | CD132 |
|---|---|---|---|
| Kd(nM) | 1.97 | - | - |
| Bmax | 0.37 | - | - |

[FIG. 4]

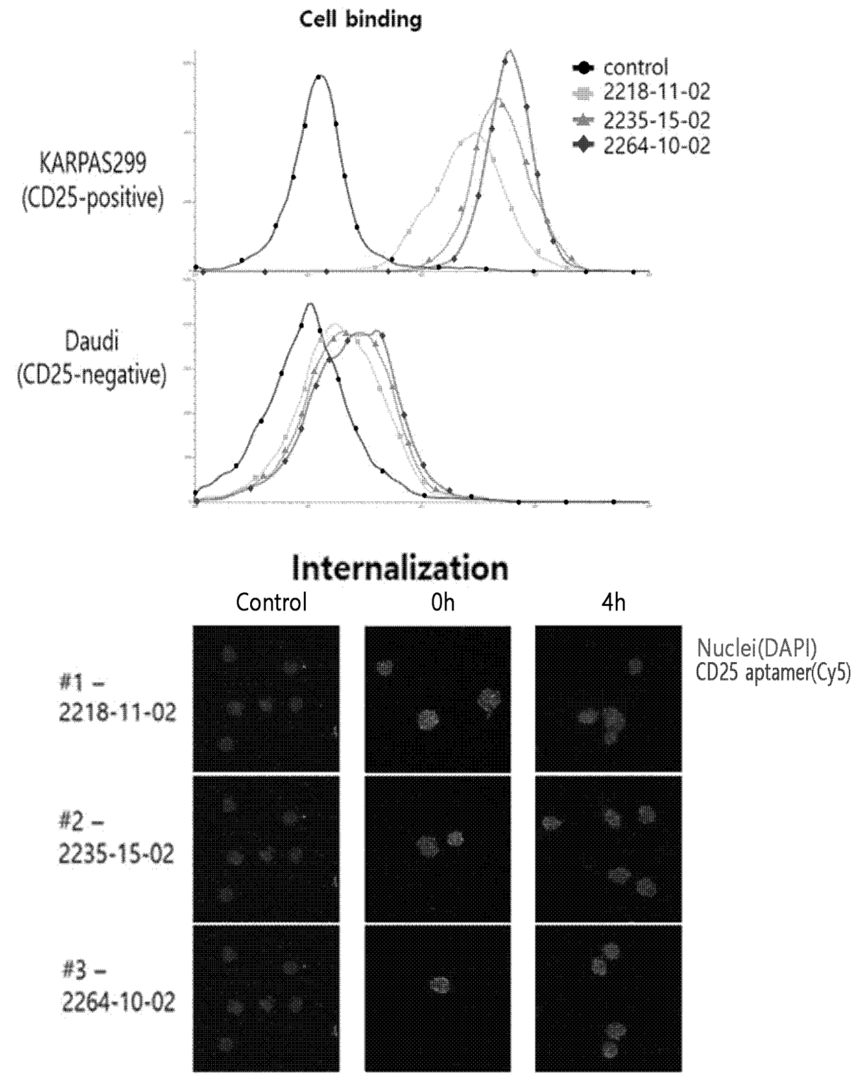
Cell binding
control
2218-11-02
2235-15-02
2264-10-02
KARPAS299
(CD25-positive)
Daudi
(CD25-negative)
Internalization
Control
0h
4h
Nuclei(DAPI)
CD25 aptamer(Cy5)
#1 –
2218-11-02
#2 –
2235-15-02
#3 –
2264-10-02

[FIG. 5]

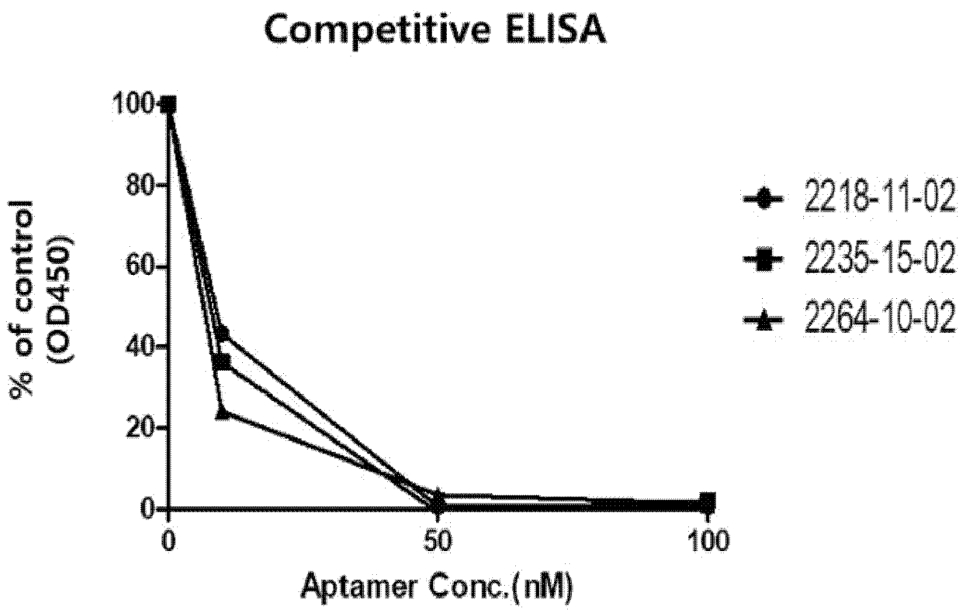
Competitive ELISA
100
80
60
40
20
0
% of control (OD450)
0
50
100
Aptamer Conc.(nM)
2218-11-02
2235-15-02
2264-10-02

[FIG. 6]

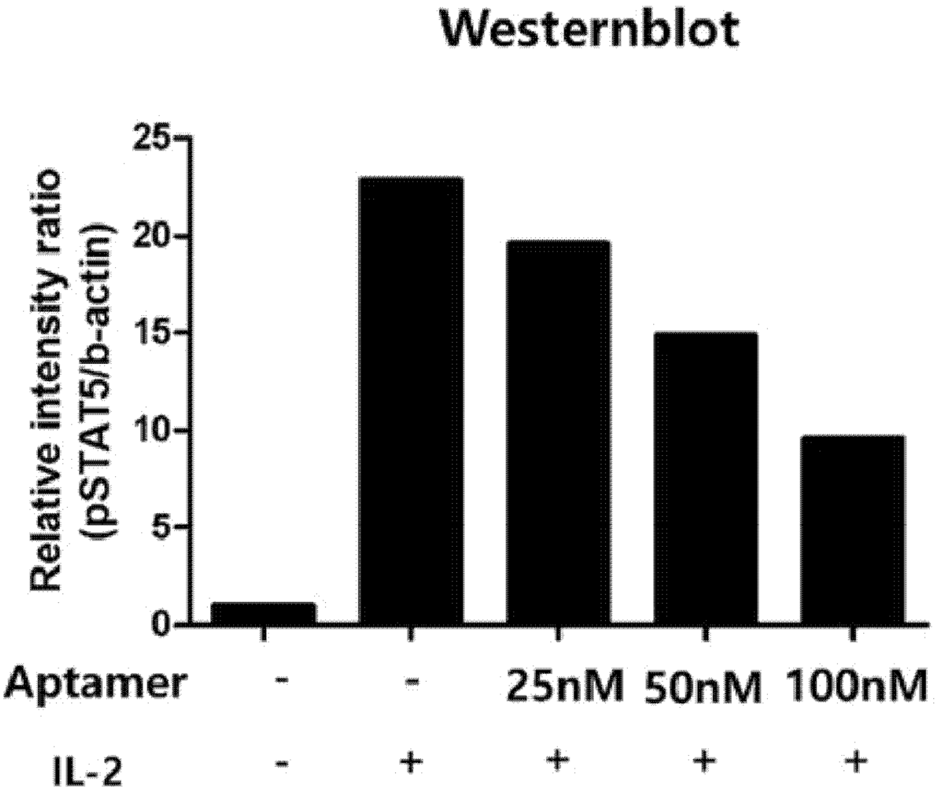
Westernblot
Relative intensity ratio (pSTAT5/b-actin)
25
20
15
10
5
0
Aptamer - - 25nM 50nM 100nM
IL-2 - + + + +

[FIG. 7]

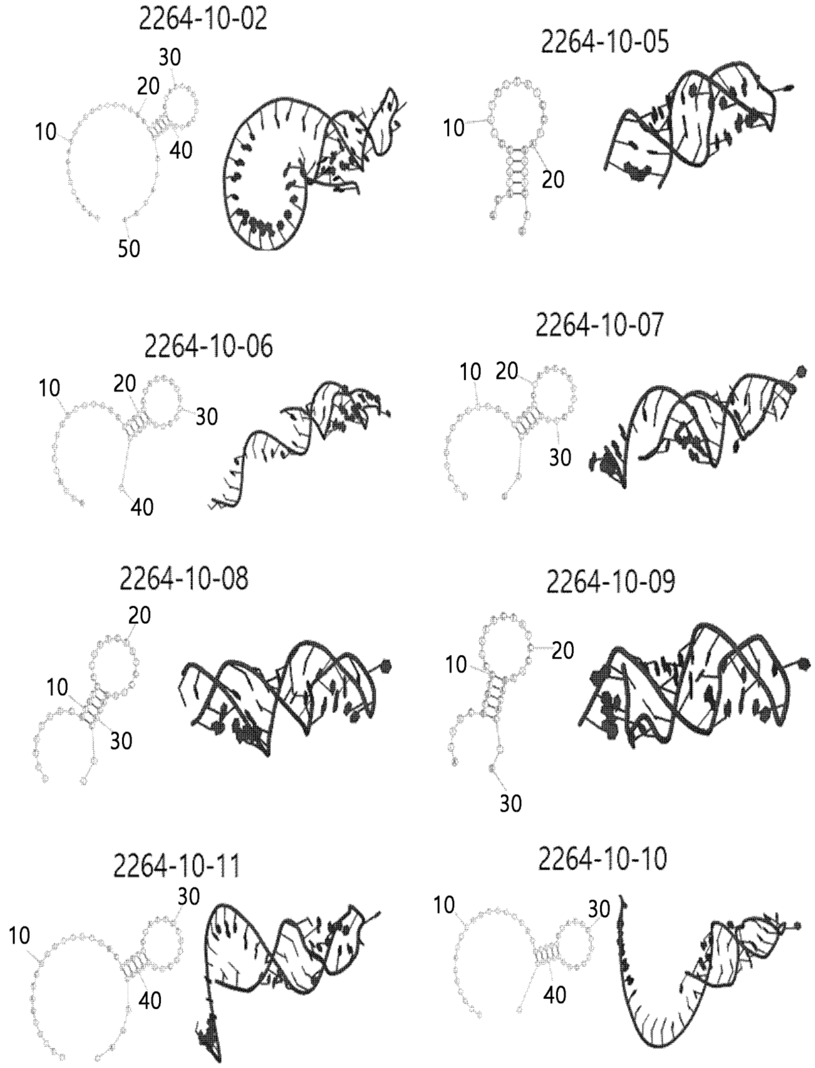
2264-10-02
30
20
10
40
50
2264-10-05
10
20
2264-10-06
10
20
30
40
2264-10-07
10
20
30
2264-10-08
20
10
30
2264-10-09
10
20
30
2264-10-11
30
10
40
2264-10-10
10
30
40

[FIG. 8]

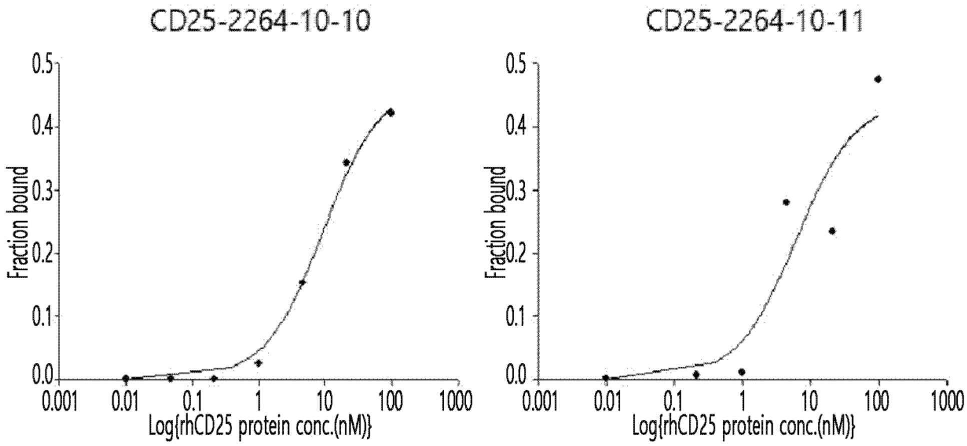


| CD25 aptamers | Mod-dU | Length(bases) | Kd(nM) | Bmax(%) | Binding potential (Bmax/Kd) |
|---|---|---|---|---|---|
| 2264-10-05 | Nap | 27 | - | - | - |
| 2264-10-06 | Nap | 40 | - | - | - |
| 2264-10-07 | Nap | 38 | - | - | - |
| 2264-10-08 | Nap | 33 | - | - | - |
| 2264-10-09 | Nap | 30 | - | - | - |
| 2264-10-10 | Nap | 46 | 8.53 | 50 | 5.86 |
| 2264-10-11 | Nap | 43 | 6.21 | 46 | 7.41 |

[FIG. 9]

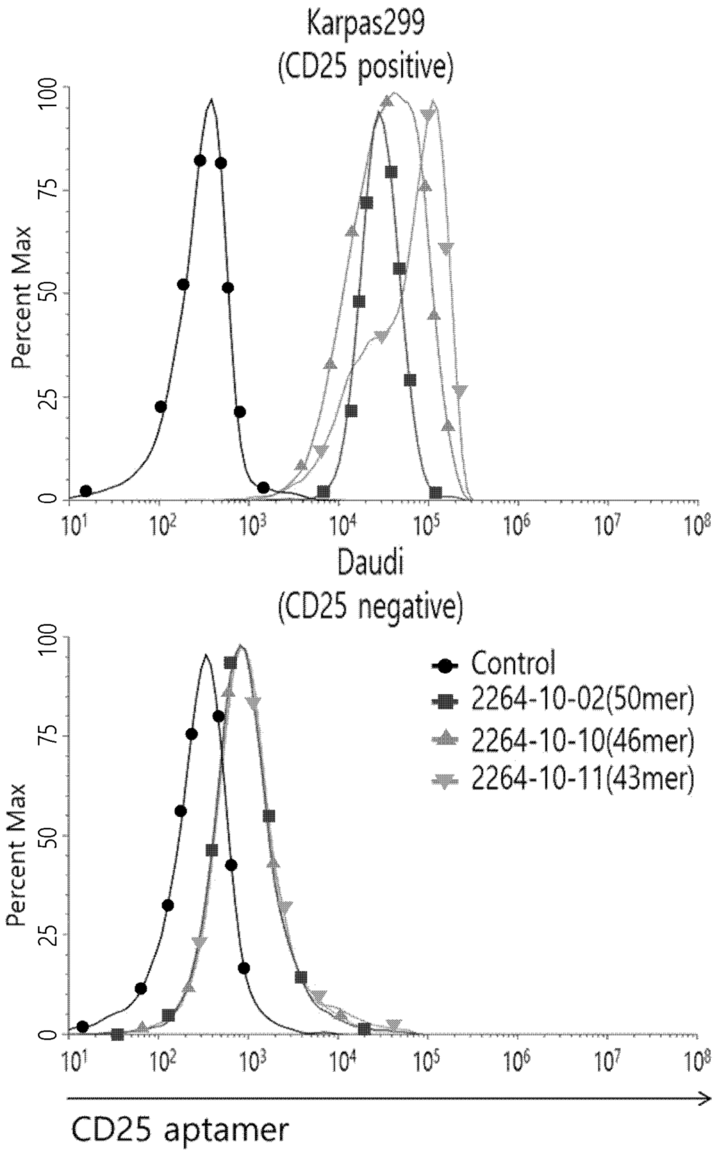
Karpas299
(CD25 positive)
Percent Max
100
75
50
25
0
$10^1$ $10^2$ $10^3$ $10^4$ $10^5$ $10^6$ $10^7$ $10^8$
Daudi
(CD25 negative)
Percent Max
Control
2264-10-02(50mer)
2264-10-10(46mer)
2264-10-11(43mer)
CD25 aptamer

[FIG. 10]

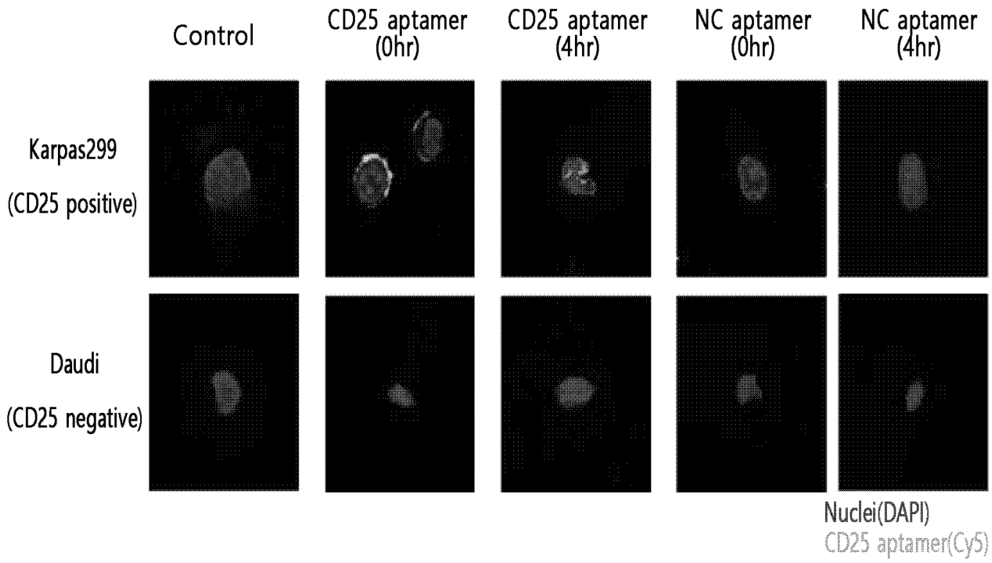
Control
CD25 aptamer (0hr)
CD25 aptamer (4hr)
NC aptamer (0hr)
NC aptamer (4hr)
Karpas299
(CD25 positive)
Daudi
(CD25 negative)
Nuclei(DAPI)
CD25 aptamer(Cy5)

[FIG. 11]

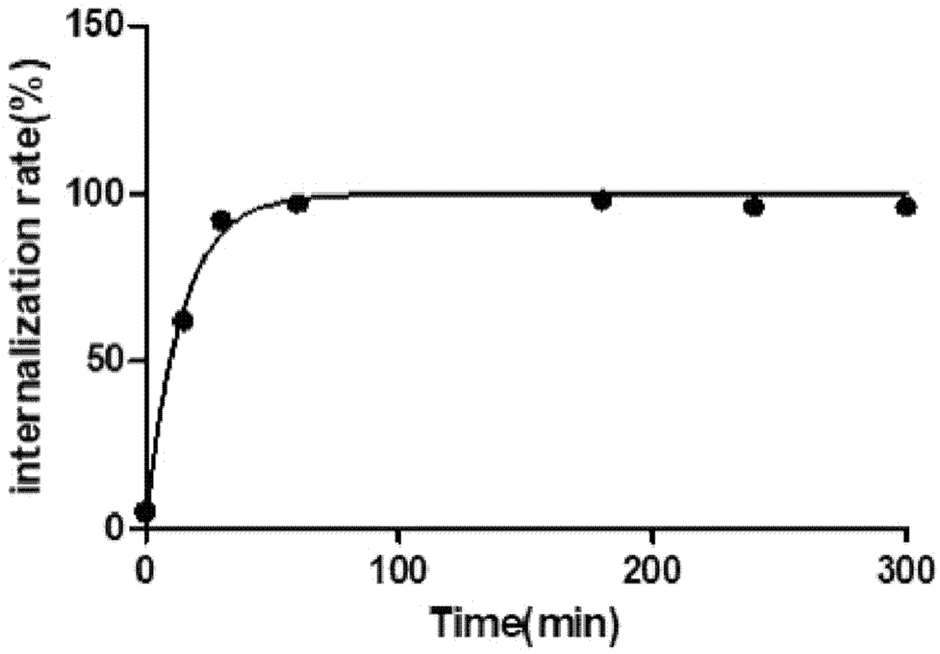
150
100
50
0
internalization rate(%)
0
100
200
300
Time(min)

[FIG. 12]

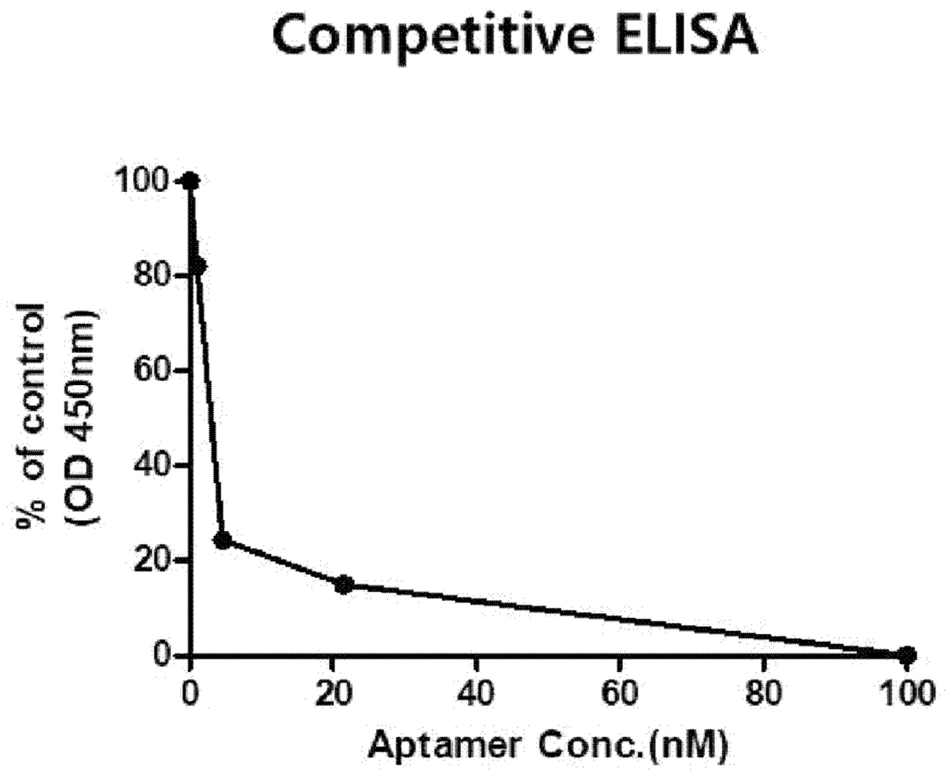
Competitive ELISA
% of control
(OD 450nm)
100
80
60
40
20
0
0
20
40
60
80
100
Aptamer Conc.(nM)

[FIG. 13]

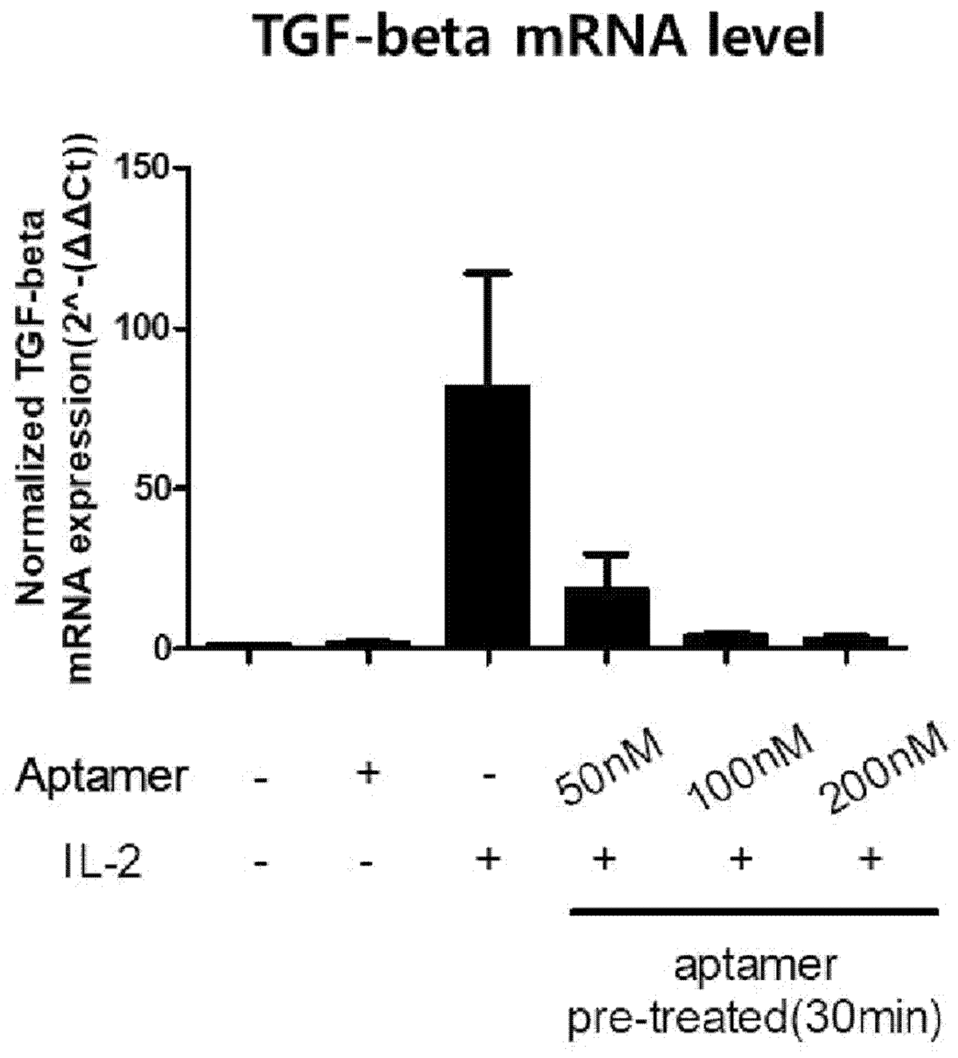
TGF-beta mRNA level
Normalized TGF-beta mRNA expression(2^-(ΔΔCt))
150
100
50
0
Aptamer - + - 50nM 100nM 200nM
IL-2 - - + + + +
aptamer pre-treated(30min)

[FIG. 14]

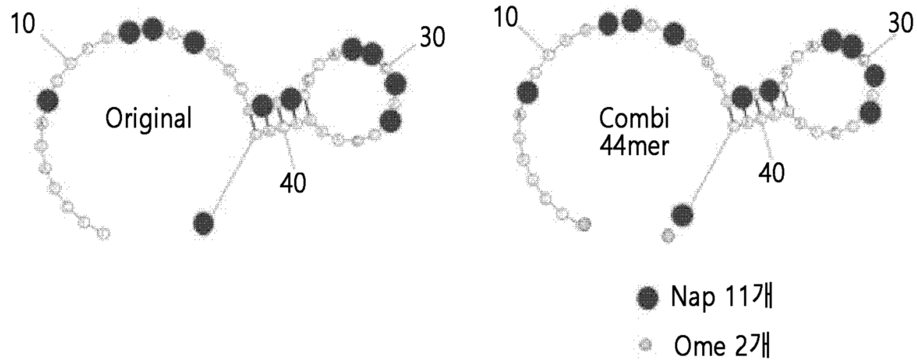

[FIG. 15]

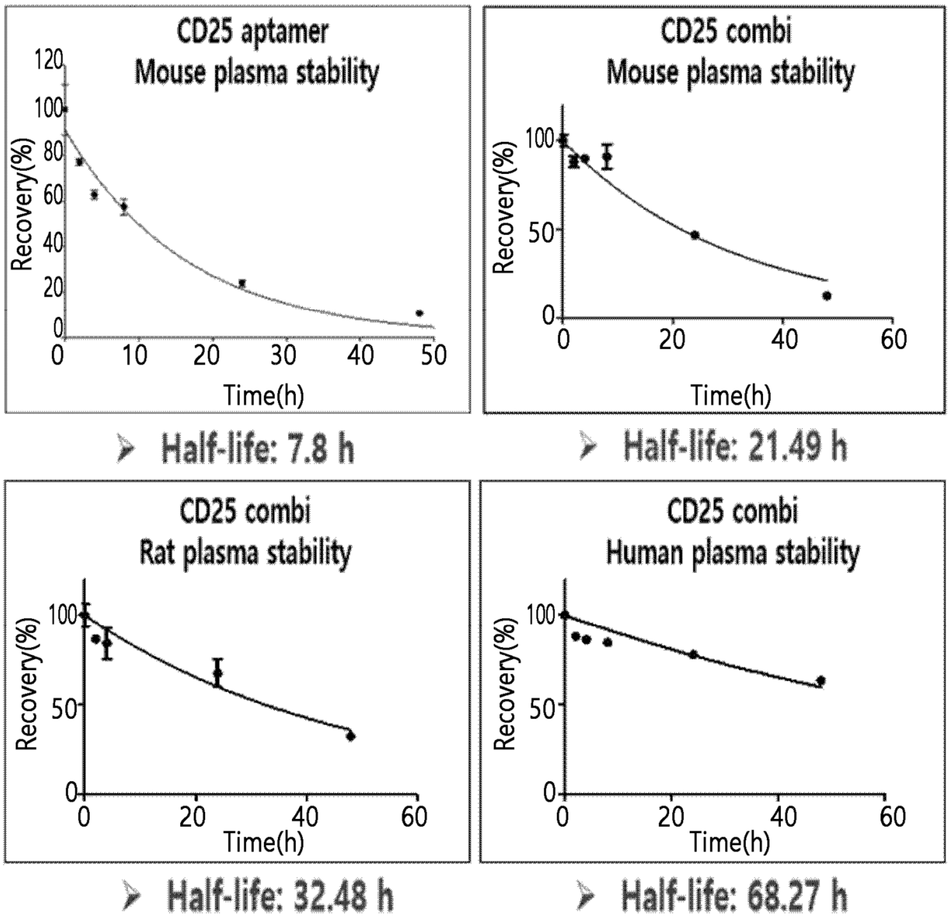
CD25 aptamer
Mouse plasma stability
Recovery(%)
Time(h)
Half-life: 7.8 h
CD25 combi
Mouse plasma stability
Recovery(%)
Time(h)
Half-life: 21.49 h
CD25 combi
Rat plasma stability
Recovery(%)
Time(h)
Half-life: 32.48 h
CD25 combi
Human plasma stability
Recovery(%)
Time(h)
Half-life: 68.27 h

[FIG. 16]

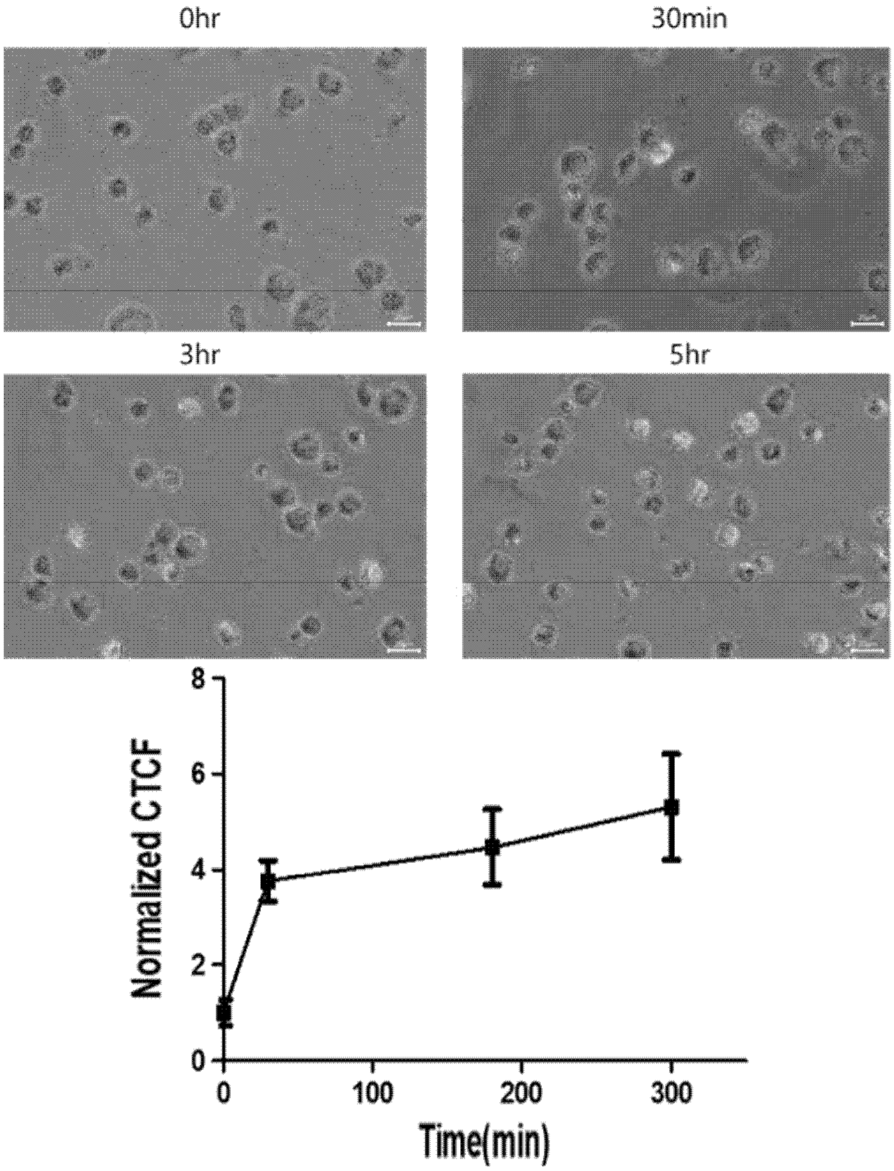
0hr
30min
3hr
5hr
Normalized CTCF
0
2
4
6
8
0
100
200
300
Time(min)

[FIG. 17]

CD25-ApDC (DApR=1)

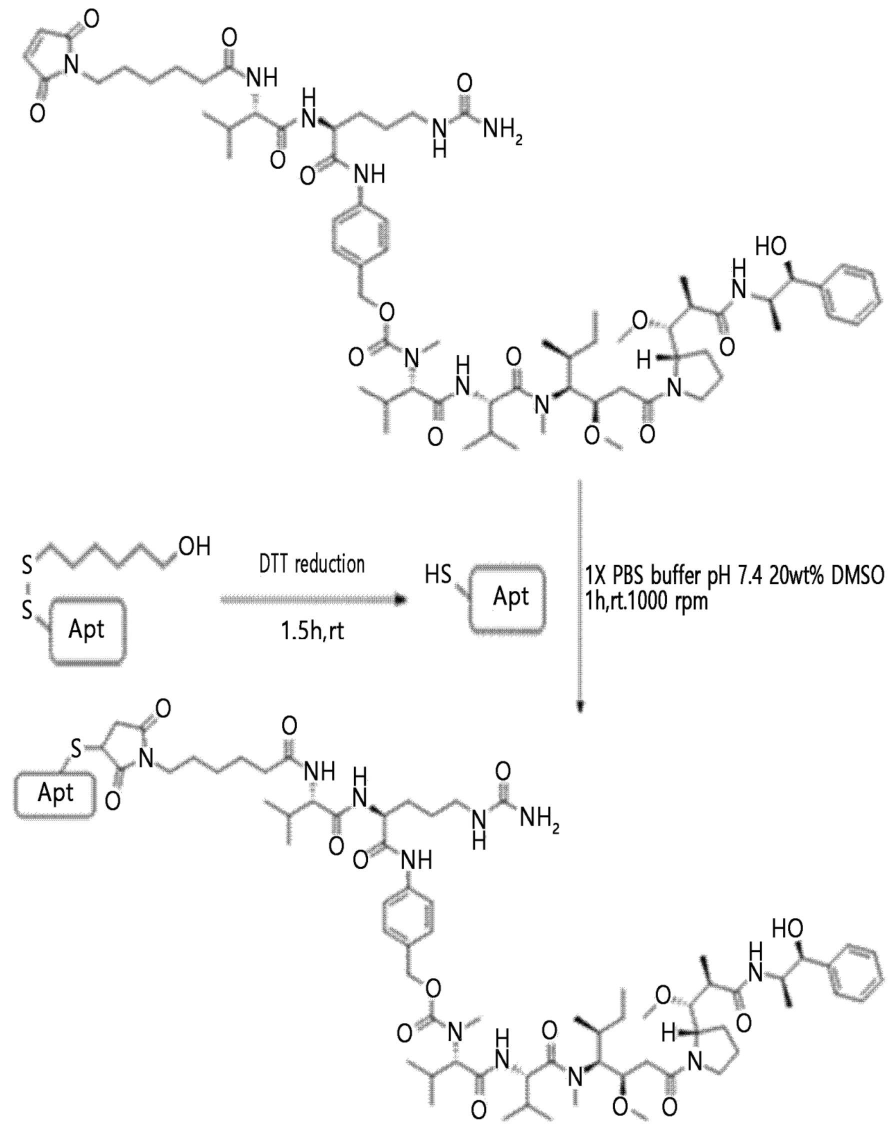

[FIG. 18]

CD25-ApDC (DApR=3)

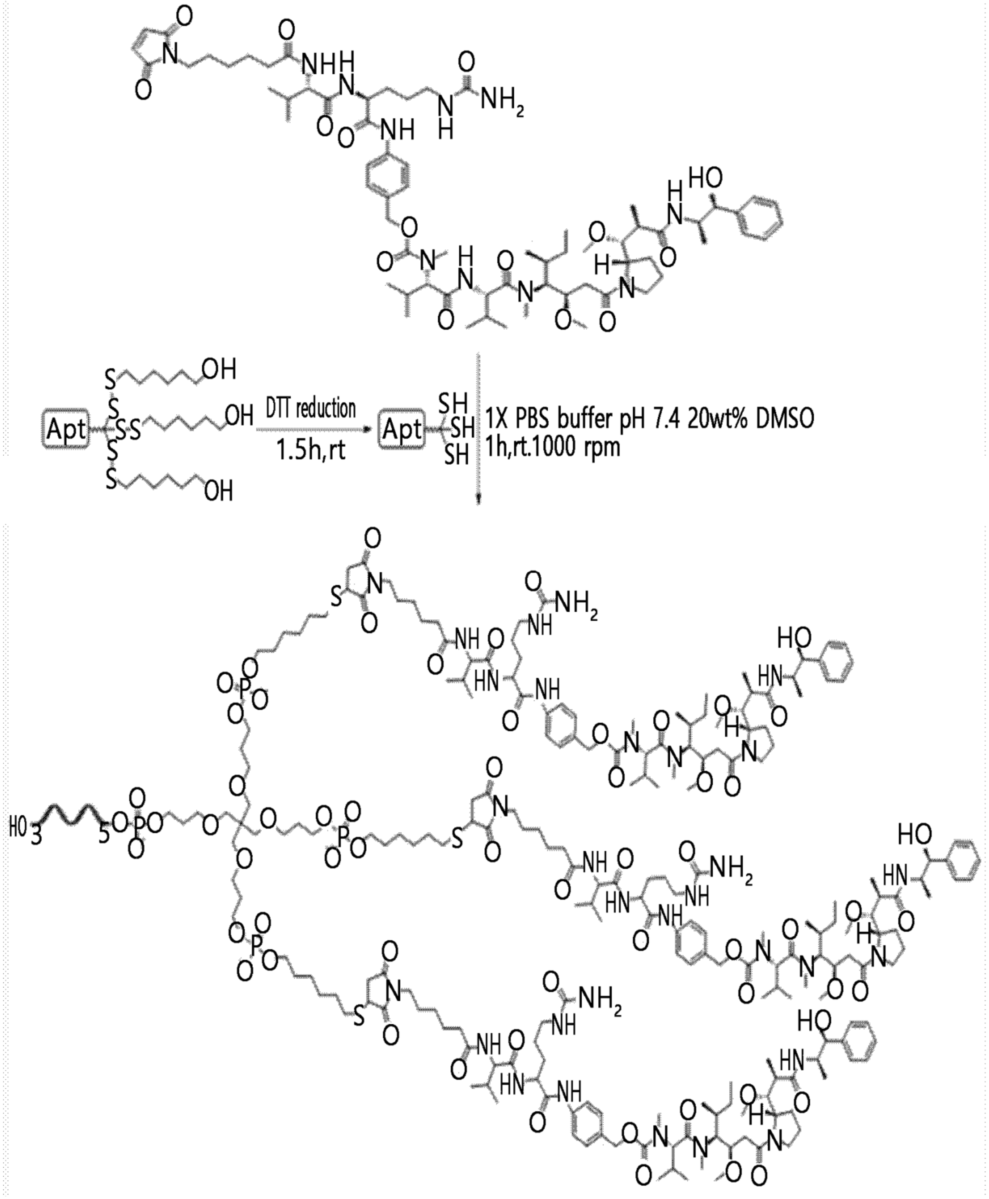

[FIG. 19]

**Karpas299**

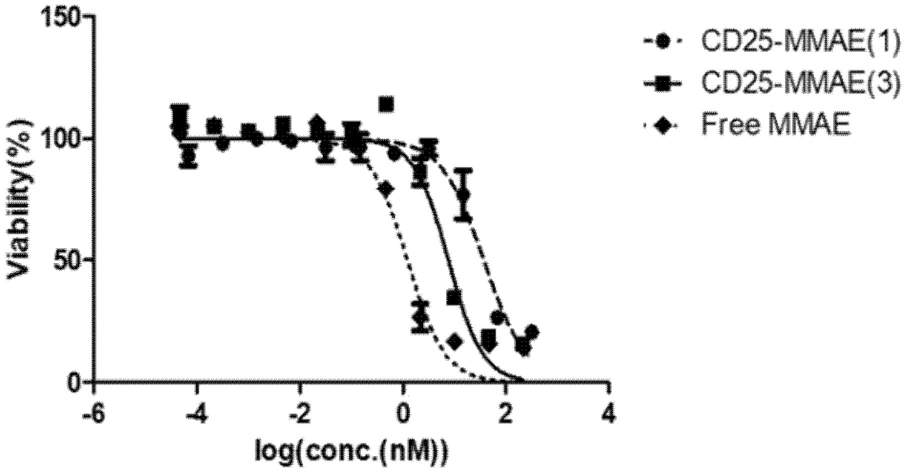


| | CD25-MMAE(1) | CD25-MMAE(3) | MMAE |
|---|---|---|---|
| IC50(nM) | 37.5 | 7.7 | 1.2 |

**Daudi**

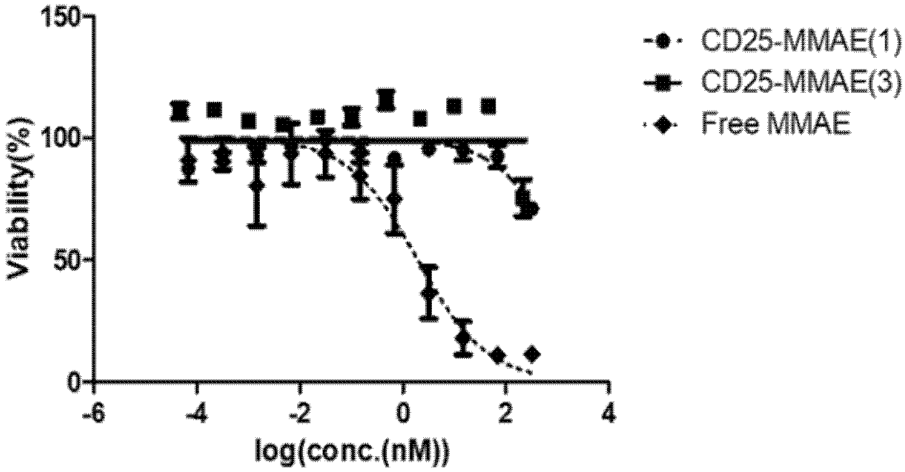


| | CD25-MMAE(1) | CD25-MMAE(3) | MMAE |
|---|---|---|---|
| IC50(nM) | - | - | 2.0 |

[FIG. 20]

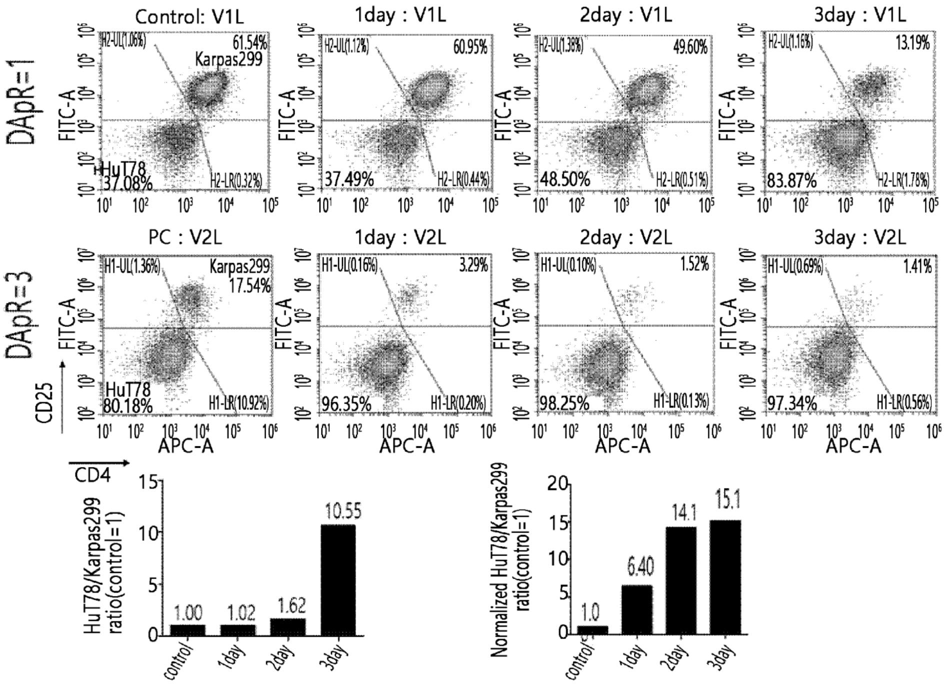
Control: V1L
1day : V1L
2day : V1L
3day : V1L
DApR=1
FITC-A
Karpas299
61.54%
HuT78
37.08%
60.95%
37.49%
49.60%
48.50%
13.19%
83.87%
PC : V2L
1day : V2L
2day : V2L
3day : V2L
DApR=3
Karpas299
17.54%
HuT78
80.18%
3.29%
96.35%
1.52%
98.25%
1.41%
97.34%
APC-A
CD25
CD4
HuT78/Karpas299 ratio(control=1)
1.00
1.02
1.62
10.55
control
1day
2day
3day
Normalized HuT78/Karpas299 ratio(control=1)
1.0
6.40
14.1
15.1

# INTERNATIONAL SEARCH REPORT

International application No.
**PCT/KR2023/017344**

## A. CLASSIFICATION OF SUBJECT MATTER

**C12N 15/115**(2010.01)i; **A61K 47/54**(2017.01)i; **A61P 35/00**(2006.01)i

According to International Patent Classification (IPC) or to both national classification and IPC

## B. FIELDS SEARCHED

Minimum documentation searched (classification system followed by classification symbols)

C12N 15/115(2010.01); A61K 39/395(2006.01); A61K 47/36(2006.01); A61K 47/42(2006.01); A61P 35/00(2006.01); C07K 14/55(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: CD25, 압타머(aptamer), 폴리뉴클레오티드(polynucleotide), IL-2, 약물 접합체(drug conjugate), 모노메틸 오리스타틴 E(monomethyl auristatin E), 링커(linker), 암(cancer)

## C. DOCUMENTS CONSIDERED TO BE RELEVANT

| Category* | Citation of document, with indication, where appropriate, of the relevant passages | Relevant to claim No. |
|---|---|---|
| | VEERAMANI, S. et al. An RNA Aptamer–Based Biomarker Platform Demonstrates High Soluble CD25 Occupancy by IL2 in the Serum of Follicular Lymphoma Patients. Cancer Immunol. Res. 2019, vol. 7, pp. 1511-1522. | |
| X | See page 1512, left column, last paragraph and page 1516, left column, fourth paragraph. | 1,3-5,12,13 |
| Y | | 14-21 |
| A | | 2,6-11 |
| | WO 2016-164745 A1 (THE METHODIST HOSPITAL SYSTEM) 13 October 2016 (2016-10-13) | |
| Y | See claims 1, 15, 21 and 25; and paragraphs [0285] and [0286]. | 14-21 |
| | SHAHDORDIZADEH, M. et al. Design, isolation and evaluation of the binding efficiency of a DNA aptamer against interleukin 2 receptor alpha, in vitro. International Immunopharmacology. 2017, vol. 53, pp. 96–104. | |
| X | See abstract; and page 98, right column, last paragraph - page 99, left column, first paragraph. | 1,3,4 |
| Y | | 17-19 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 March 2024** | **11 March 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

INTERNATIONAL SEARCH REPORT

International application No.
**PCT/KR2023/017344**

C. DOCUMENTS CONSIDERED TO BE RELEVANT

| Category* | Citation of document, with indication, where appropriate, of the relevant passages | Relevant to claim No. |
|---|---|---|
| A | US 2019-0136240 A1 (UNIVERSITY OF IOWA RESEARCH FOUNDATION) 09 May 2019 (2019-05-09)<br>See entire document. | 1-21 |
| A | KR 10-2022-0168607 A (KIM, Sung Chun) 26 December 2022 (2022-12-26)<br>See entire document. | 1-21 |
| X | 신문기사. 압타머사이언스, `CD25 타깃 ApDC` KDDF 과제선정. 바이오스펙테이터. 02 November 2022, pp. 1-2, non-official translation (Newspaper article. Aptamer Sciences, Inc., 'CD25 Target ApDC' Selected as a KDDF Task. BioSpectator). Retrieved from the Internet <URL:http://m.biospectator.com/view/news_view.php?varAtcId=17567>.<br>See pages 1-2.<br>※ This document is a known document declaring `non-prejudicial disclosures or exceptions to lack of novelty` by the applicant. | 1-21 |
| X | LEE, D. et al. Abstract 6415: CD25-targeted aptamer-drug conjugate (CD25-ApDC) depletes and blocks regulatory T cells selectivel. Cancer Res. 04 April 2023, vol. 83, 7_Supplement, document no. 6415, p. 1.<br>See page 1.<br>※ This document is a known document declaring 'non-prejudicial disclosures or exceptions to lack of novelty' by the applicant. | 1-21 |

Form PCT/ISA/210 (second sheet) (July 2022)

**INTERNATIONAL SEARCH REPORT**

International application No.
**PCT/KR2023/017344**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/KR2023/017344**

| Patent document cited in search report | Publication date (day/month/year) | Patent family member(s) | Publication date (day/month/year) |
|---|---|---|---|
| WO 2016-164745 A1 | 13 October 2016 | US 10683506 B2 | 16 June 2020 |
| | | US 2018-0073027 A1 | 15 March 2018 |
| US 2019-0136240 A1 | 09 May 2019 | US 10934549 B2 | 02 March 2021 |
| | | US 2021-0180067 A1 | 17 June 2021 |
| KR 10-2022-0168607 A | 26 December 2022 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **Y. Y. SETIADY et al.** *Eur. J. Immunol.*, 2010, vol. 40 (3), 780-786
- **M. ZHANG. et al.** *Cancer Res.*, 2006, vol. 66 (16), 8227
- **PEARSON et al.** *PNAS*, 1988, vol. 85 (8), 2444-2448
- **NEEDLEMAN** ; **WUNSCH**. *J. Mol. Biol.*, 1970, vol. 48, 443-453
- **RICE et al.** EMBOSS: The European MolecuLar Biology Open Software Suite. *Trends Genet.*, 2000, vol. 16, 276-277
- **DEVEREUX et al.** *Nucleic Acids Research*, 1984, vol. 12, 387-395
- **ATSCHUL et al.** *J. Mol. Biol.*, 1990, vol. 215 (3), 403-410
- **MARTIN J. BISHOP**. Guide to Huge Computers. Academic Press, 1994
- **CARILLO et al.** *SIAM J. Appl. Math.*, 1988, vol. 48 (5), 1073-1082
- **NEEDLEMAN et al.** *J Mol Biol.*, 1970, vol. 48 (3), 443-453
- **SMITH** ; **WATERMAN**. *Adv. Appl. Math.*, 1981, vol. 2 (4), 482-489
- **GRIBSKOV et al.** *Nucl. Acids Res.*, 1986, vol. 14 (16), 6745-6763
- Atlas Of Protein Sequence And Structure. National Biomedical Research Foundation, 1979, 353-358
- **J. SAMBROOK et al.** MolecuLar Cloning, A Laboratory Manual. Cold Spring Harbor Laboratory press, 1989
- **F.M. AUSUBEL et al.** Current Protocols in MolecuLar Biology. John Wiley & Sons, Inc.
- **SETIADY et al.** *Eur. J. Immunol.*, 2010, vol. 40 (3), 780-786
- **ZHANG et al.** *Cancer Res.*, 2006, vol. 66 (16), 8227